# EUROPEAN PATENT APPLICATION

(11) **EP 3 928 791 A1**
(43) Date of publication of application: **29.12.2021**
(21) Application number: 20758592.8
(22) Date of filing: 21.02.2020
(51) Int. Cl.: A61K 39/395, A61K 45/00, A61K 48/00, A61P 1/16, A61P 9/00, A61P 9/10, A61P 11/00, A61P 13/12, A61P 43/00, A61P 17/00, A61K 31/7088, A61K 31/7105, A61K 31/711, A61K 31/713

(54) **PREVENTION OR TREATMENT OF FIBROTIC DISEASE**

(30) Priority: 22.02.2019 JP 2019030791
(71) Applicant: Kyushu University, National University Corporation, Nishi-ku Fukuoka-shi Fukuoka 819-0395 (JP)
(72) Inventor: NAKAYA, Michio, Fukuoka-shi, Fukuoka 819-0395 (JP); KUROSE, Hitoshi, Fukuoka-shi, Fukuoka 819-0395 (JP)
(74) Representative: Vossius & Partner Patentanwälte Rechtsanwälte mbB
(86) International application number: PCT/JP2020/007072
(87) International publication number: WO 2020/171206

(57) **Abstract**

A modality for preventing or treating fibrotic diseases by identifying a marker protein for myofibroblasts is provided.

The present invention relates to prophylactic or therapeutic agents for fibrotic diseases, which contain an inhibitor of GPR176 as an active ingredient.

## Description

### TECHNICAL FIELD

The present invention relates to a pharmaceutical composition for preventing or treating fibrotic diseases. In particular, the invention relates to agents and nucleic acid molecules that prevent or treat fibrotic diseases, as well as methods of screening for them, and kits therefor.

### BACKGROUND TECHNOLOGY

Fibrosis represents a state in which extracellular matrixes such as a collagen are excessively produced in living tissues. Fibrosis is known to cause aggravation of the pathological conditions of various organs such as heart, liver, kidney, and lung. However, there have been few effective therapeutic drugs for fibrotic diseases conventionally, and the development for such drugs is required.

It is known that fibrosis in tissues is driven by a group of cells called myofibroblasts, which produce collagens and the like. It is also known that myofibroblasts are rarely present when the tissue is normal, and are created by differentiation of various cells, which is triggered by inflammation.

In the past, proteins such as α-Smooth Muscle Actin (α-SMA) have been known as markers for myofibroblasts; see, for example,

Patent Document 1.

### CITATION LIST

### PATENT DOCUMENTS

Patent Document 1: Japanese Patent Publication (Kokai) No. 2004-81122

### SUMMARY OF THE INVENTION

### PROBLEMS TO BE SOLVED BY THE INVENTION

However, α-SMA may not be necessarily deemed to be a myofibroblast-specific marker protein in a strict sense, for the reason that it is strongly expressed in vascular smooth muscle, and for its cell specificity, its expression time, and the like. Therefore, identification of reliable marker proteins for myofibroblast is a bottleneck in research for myofibroblasts.

The purpose of the present invention is to identify a marker protein for myofibroblasts and to provide a modality for preventing or treating fibrotic diseases.

### MEANS TO SOLVE PROBLEMS

G Protein-Coupled Receptor 176 (hereinafter referred to as GPR176) is registered as one of the orphan receptors in NCBI (https://www.ncbi.nlm.nih.gov/gene/?term=GPR176). So far, GPR176 has been identified merely as a Gz-coupled type orphan G protein-coupled receptor that provides the set of circadian behavior pace (NATURE COMMUNICATIONS | 7:10583 | DOI: 10.1038/ncomms10583 | Published 17 Feb 2016.).

The present inventors have earnestly studied aiming at preventing or treating fibrotic diseases, and, as a result, have found that GPR176 is strongly associated with fibrosis of organs. Further, having pushed forward a study, the present inventors have found that the inhibition or suppression of the GPR176 expression and function facilitates to reduce or suppress fibrosis, thereby completing the present invention.

Specifically, the present invention encompasses the following aspects:
<Pharmaceutical composition for preventing or treating fibrotic diseases>
   [1] A pharmaceutical composition for preventing or treating fibrotic diseases, which comprises an inhibitor of G protein-coupled receptor 176 (GPR176) as an active ingredient;
   [2] The pharmaceutical composition according to [1], wherein the inhibitor of GPR176 is an inhibitor of the GPR176 expression or an inhibitor of the GPR176 function;
   [3] The pharmaceutical composition according to [2], wherein the inhibitor of GPR176 is an inhibitor of the GPR176 expression;
   [4] The pharmaceutical composition according to [2], wherein the inhibitor of the GPR176 expression is a substance that inhibits the expression of the gene or nucleic acid represented by any one of (a) to (c):
      (a) the GPR176 gene set forth in any one of SEQ ID NOs: 2, 4 or 6 of the Sequence Listing,
      (b) a nucleic acid that comprises a base sequence containing a deletion, a substitution or an addition of one or several bases in the GPR176 gene set forth in any one of SEQ ID NOs: 2, 4 or 6 of the Sequence Listing, and
      (c) a nucleic acid that hybridizes under stringent conditions with a base sequence complementary to the GPR176 gene set forth in any one of SEQ ID NOs: 2, 4 or 6 of the Sequence Listing;
   [5] The pharmaceutical composition according to [4], wherein the substance that inhibits the expression of the gene or nucleic acid is selected from the group consisting of an siRNA, an antisense and a ribozyme;
   [6] The pharmaceutical composition according to [2], wherein the inhibitor of GPR176 is an inhibitor of the GPR176 function;
   [7] The pharmaceutical composition according to claim [6], wherein the inhibitor of the GPR176 function is a substance specifically binding to the GPR176 protein;
   [8] The pharmaceutical composition according to [7], wherein the substance specifically binding to the GPR176 protein is selected from the group consisting of an antibody, an antibody fragment and an aptamer;
   [9] The pharmaceutical composition according to [8], wherein the antibody fragment is selected from the group consisting of Fv, Fab and scFv;
<Nucleic acid, etc.>
   [10] A nucleic acid selected from the group consisting of an siRNA, an antisense and a ribozyme, which inhibits the expression of:
      (a) the GPR176 gene set forth in any one of SEQ ID NOs: 2, 4 or 6 of the Sequence Listing,
      (b) a nucleic acid that comprises a base sequence containing a deletion, a substitution or an addition of one or several bases in the GPR176 gene set forth in any one of SEQ ID NOs: 2, 4 or 6 of the Sequence Listing, or
      (c) a nucleic acid that hybridizes under stringent conditions with a base sequence complementary to the GPR176 gene set forth in any one of SEQ ID NOs: 2, 4 or 6 of the Sequence Listing;
   [11] A vector comprising the gene or nucleic acid according to [10];
   [12] A cell containing the vector according to [11];
<Screening method>
   [13] A method of screening for an inhibitor of GPR176, which comprises confirming that the expression of a gene or a nucleic acid is inhibited, which is characterized by the use of said gene or said nucleic acid represented by any one of (a) to (c):
      (a) the GPR176 gene set forth in any one of SEQ ID NOs: 2, 4, 6 or 8 of the Sequence Listing,
      (b) a nucleic acid that comprises a base sequence containing a deletion, a substitution or an addition of one or several bases in the GPR176 gene set forth in any one of SEQ ID NOs: 2, 4, 6 or 8 of the Sequence Listing, or
      (c) a nucleic acid that hybridizes under stringent conditions with a base sequence complementary to the GPR176 gene set forth in any one of SEQ ID NOs: 2, 4, 6 or 8 of the Sequence Listing, or
         the use of a cell into which the gene or nucleic acid has been introduced;
   [14] The method of screening for an inhibitor of GPR176 according to [13], which comprises;
      (1) preparing a candidate compound,
      (2) contacting said candidate compound to a cell containing the gene or nucleic acid represented by any one of:
         (a) the GPR176 gene set forth in any one of SEQ ID NOs: 2, 4, 6 or 8 of the Sequence Listing,
         (b) a nucleic acid that comprises a base sequence containing a deletion, a substitution or an addition of one or several bases in the GPR176 gene set forth in any one of SEQ ID NOs: 2, 4, 6 or 8 of the Sequence Listing, or
         (c) a nucleic acid that hybridizes under stringent conditions with a base sequence complementary to the GPR176 gene set forth in any one of SEQ ID NOs: 2, 4, 6 or 8 of the Sequence Listing, and
      (3) determining whether or not said candidate compound suppresses the expression of said gene or nucleic acid;
   [15] The method according to [13] or [14], which comprises using the GPR176 gene set forth in any one of SEQ ID NOs: 2, 4 or 6 of the Sequence Listing;
   [16] A method of screening for a substance that inhibits the function of a protein, characterized by the use of said protein, which is the GPR176 protein having the amino acid sequence set forth in any one of SEQ ID NOs: 1, 3, 5 or 7 of the Sequence Listing, or a variant GPR176 protein which contains a substitution, a deletion or an addition of one or several amino acid residues in the GPR176 protein, and which has the same activity as said GPR176 protein;
   [17] A method of screening for a substance that inhibits the function of the GPR176 protein, which comprises;
      (1) preparing a candidate compound,
      (2) contacting said candidate compound to the GPR176 protein having the amino acid sequence set forth in any one of SEQ ID NOs: 1, 3, 5 or 7 of the Sequence Listing, or a variant GPR176 protein which contains a substitution, a deletion or an addition of one or several amino acid residues in the GPR176 protein, and which has the same activity as said GPR176 protein, or to a cell expressing said GPR176 protein or said variant GPR176 protein, and
      (3) determining whether or not said candidate compound inhibits the function of said GPR176 protein or said variant GPR176 protein;
   [18] The method according to claim 16 or 17, which comprises using the GPR176 protein having the amino acid sequence set forth in any one of SEQ ID NOs: 1, 3 or 5 of the Sequence Listing, or a variant GPR176 protein which contains a substitution, a deletion or an addition of one or several amino acid residues in the GPR176 protein, and which has the same activity as said GPR176 protein;
   [19] A method of screening for a prophylactic or therapeutic agent for fibrotic diseases, which comprises a step of culturing myofibroblasts in the presence of a candidate compound, a step of quantifying the expression level of the mRNA of the GPR176 gene or the GPR176 protein in the cultured myofibroblasts, and a step of judging the candidate compound to be a prophylactic or therapeutic agent for fibrotic diseases when the quantified expression level of the mRNA of said GPR176 gene or said GPR176 protein is decreased as compared with that of the control;
<Biomarkers, etc.>
   [20] A method of determining the severity in fibrotic diseases, which comprises;
      (a10) a step of measuring the amount of GPR176 on myofibroblasts of a subject (test biomarker amount),
      (b10) a step of comparing the amount of the test biomarker with the amount of GPR176 on myofibroblasts of a healthy subject (control biomarker amount), and
      (c10) a step of determining that fibrotic diseases become serious when the test biomarker amount is larger than the control biomarker amount;
   [21] A biomarker that is GPR176 of myofibroblasts which enables to determine the severity in fibrotic diseases;
<Kit>
   A kit for detecting myofibroblasts, which comprises a primer set for amplifying the cDNA of GPR176, a probe that specifically hybridizes with the mRNA of GPR176, or a substance specifically binding to the GPR176 protein.

### EFFECT OF INVENTION

According to the present invention, it is possible to identify a marker protein for myofibroblasts, and to provide a modality for preventing or treating fibrotic diseases.

### BRIEF DESCRIPTION OF DRAWINGS

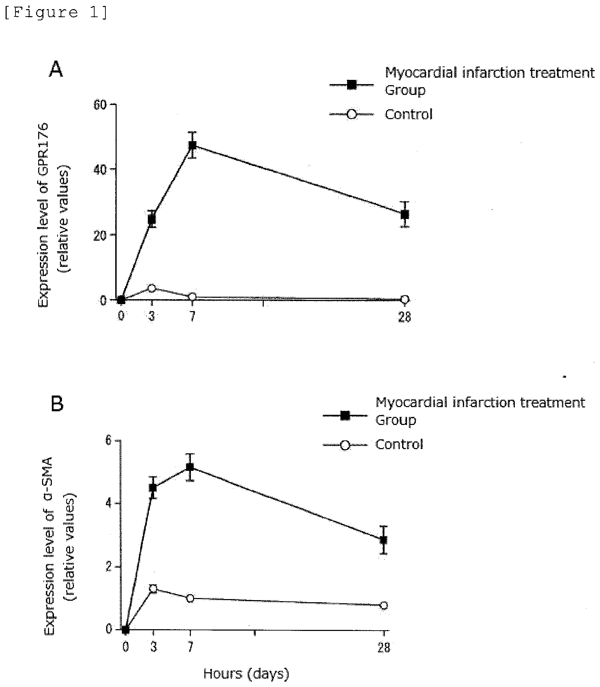

Figure 1A is a graph showing the results of the mRNA expression level of GPR176 measured in the heart tissues of the myocardial infarction model mice and the control mice. Figure 1B is a graph showing the results of the mRNA expression level of α-SMA measured in the heart tissues of the myocardial infarction model mice and the control mice.

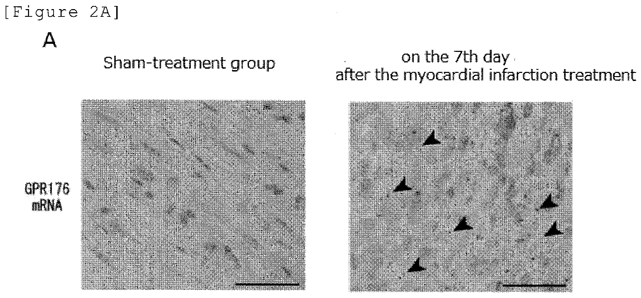

Figure 2A is photographs showing the results of in situ hybridization evaluating the GPR176 mRNA in the heart sections of the WT (wild-type) mice of the sham treatment, or on the 7th day after the myocardial infarction treatment. The nuclei were counter-stained with hematoxylin. Black arrows indicate the GPR176 mRNA. Scale bar: 50 µm.

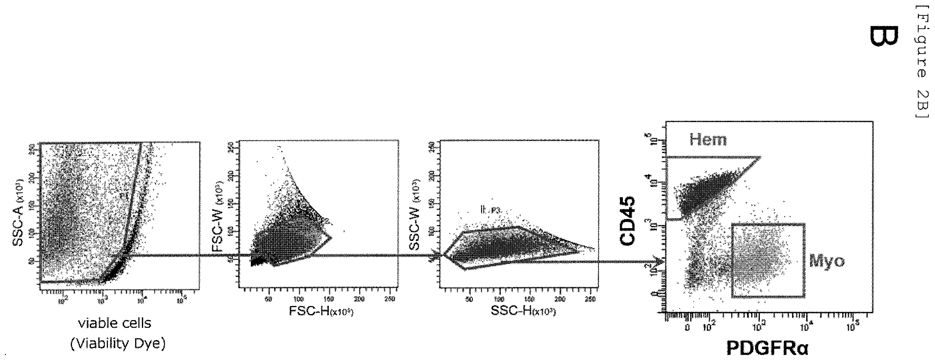

Figure 2B shows the setting of the gates in FACS for sorting leukocytes (Hem) and myofibroblasts (Myo) of the heart after the myocardial infarction treatment. The cells containing the leukocytes and the myofibroblasts were isolated from the heart of the WT mice on the third day after the myocardial infarction treatment. The cells immediately isolated were stained with the anti-PDGFR-α antibody and the anti-CD45 antibody, and analyzed by FACS.

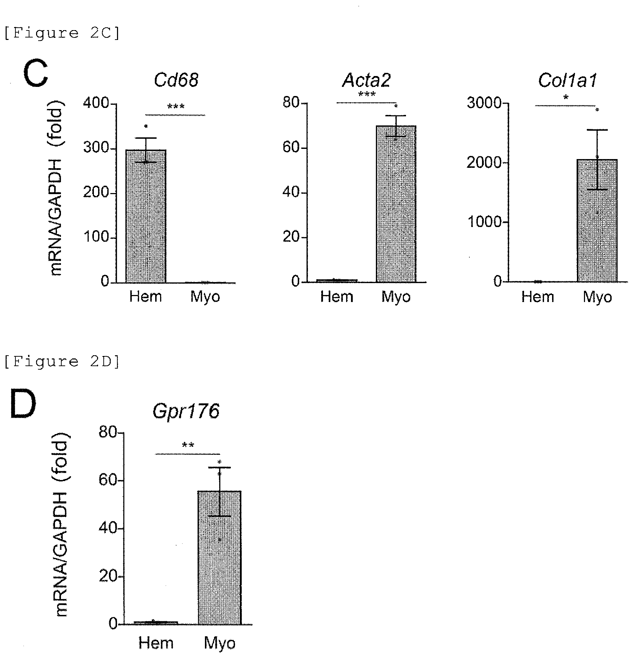

Figure 2C is graphs showing the mRNA expression levels of the indicated genes (Cd68, Acta2 and Colla1) in the cells sorted as the leukocytes (Hem) and the myofibroblasts (Myo). Cd68 and Colla1/Acta2 were used as markers for macrophages and myofibroblasts, respectively. n = 3. GAPDH was used as an internal control. * P <0.05, ** P <0.01, *** P <0.001, unpaired two-sided student's t-test.
Figure 2D is a graph showing the mRNA expression level of GPR176 in the cells sorted as the leukocytes (Hem) and the myofibroblasts (Myo). n = 3. GAPDH was used as an internal control. * P <0.05, ** P <0.01, *** P <0.001, unpaired two-sided student's t-test.

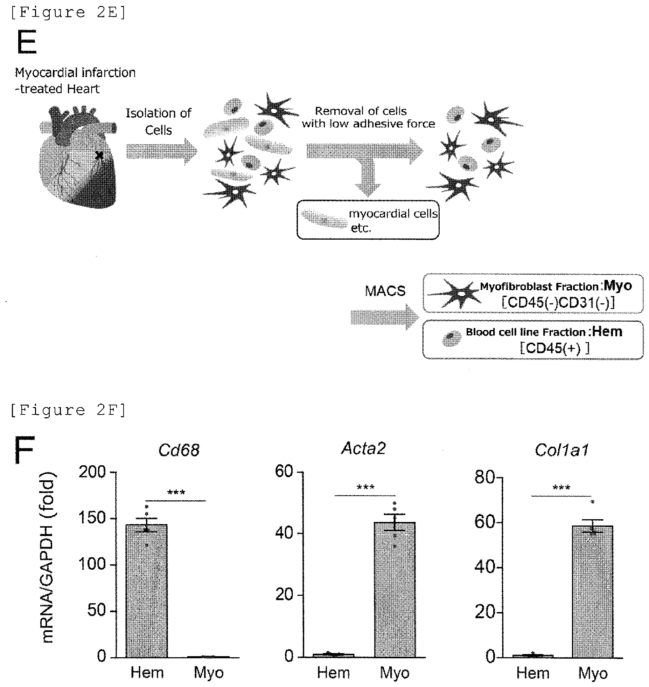

Figure 2E is a schematic diagram showing the cell sorting from a heart treated with myocardial infarction using MACS.
Figure 2F is graphs showing the mRNA expression levels of the indicated genes (Cd68, Acta2 and Colla1) in the cells sorted as the leukocytes (Hem) and the myofibroblasts (Myo). Cd68 and Collal/Acta2 were used as markers for macrophages and myofibroblasts, respectively. n = 5. GAPDH was used as an internal control. * P <0.05, ** P <0.01, *** P <0.001, unpaired two-sided student's t-test.

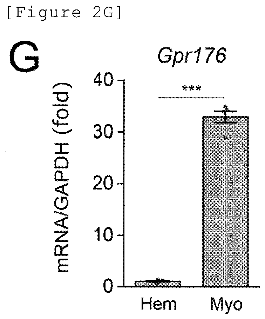

Figure 2G is a graph showing the mRNA expression level of GPR176 in the cells sorted as the leukocytes (Hem) and the myofibroblasts (Myo). n = 5. GAPDH was used as an internal control. * P <0.05, ** P <0.01, *** P <0.001, unpaired two-sided student's t-test.

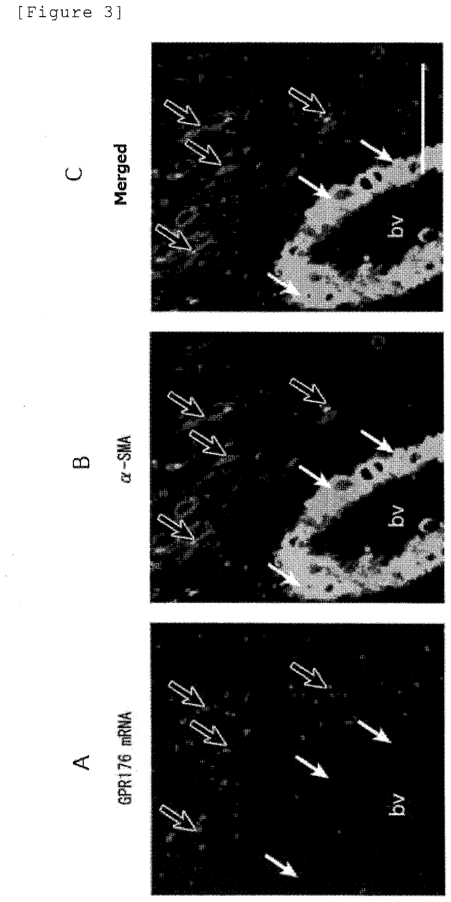

Figure 3: Figures 3A to 3C are fluorescence micrographs showing the results of the in situ hybridization (GPR176 mRNA) and the immuno-staining (α-SMA).

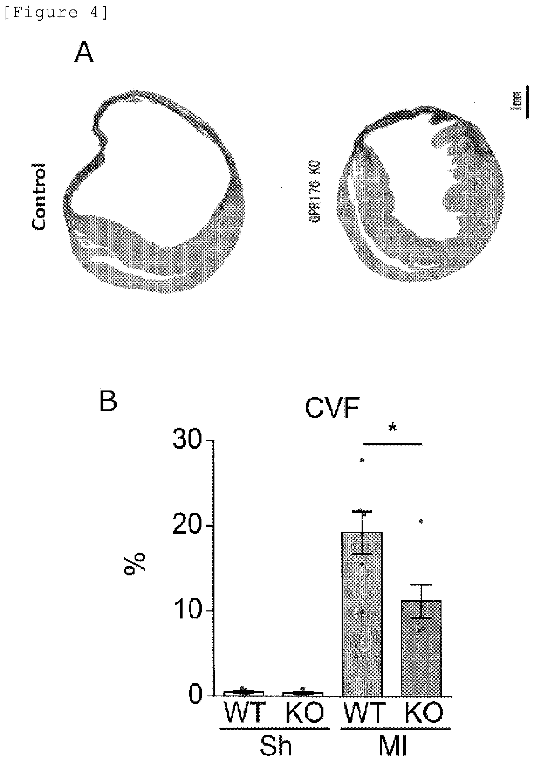

Figure 4A is photographs showing the results of the picrosirius red staining of the paraffin-embedded ventricular sections from the wild-type (WT) mice and the GPR176-KO mice 28 days after the myocardial infarction treatment. Scale bar, 1 mm. Figure 4B: A graph showing the quantitative data on the Collagen Volume Fraction (CVF) of the WT and the GPR176-KO mice 28 days after the myocardial infarction treatment. CVS was estimated by determining the collagen deposit area. WT; n = 6, GPR176 KO; n = 6. Error bars represent the mean ± SEM. Figure 4B: * P <0.05, unpaired two-sided student's t-test.

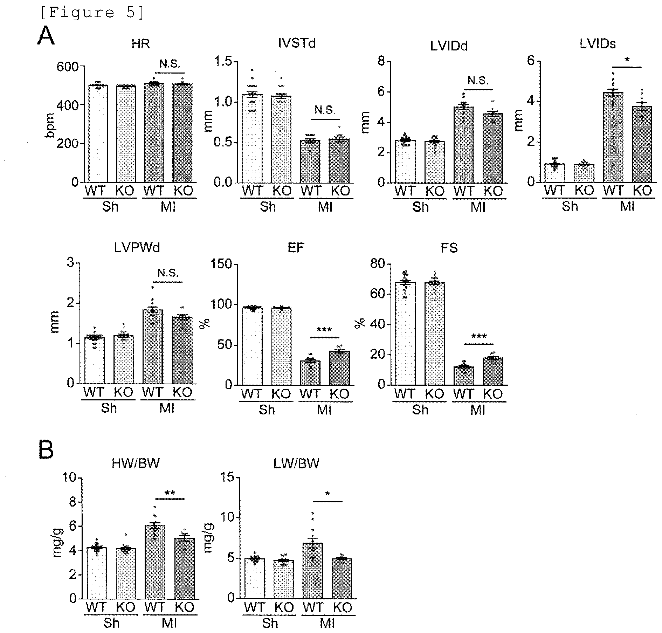

Figure 5 shows that the GPR176 deficiency improves the cardiac function after the infarction treatment. Figure 5A: Graphs showing the results of the echocardiographic analysis of the hearts of the wild-type (WT) mice and the GPR176-KO mice 28 days after the myocardial infarction treatment. HR; Heart rate, IVSTd; Diastolic interventricular septal thickness, LVIDd; Left ventricular end diastolic inner diameter, LVID; Left ventricular end systolic inner diameter, LVPWd; Left ventricular posterior wall diastole, EF; Ejection fraction, FS; Left ventricular diameter Shortening rate. WT-Sh (Sham treatment); n = 18, KO-Sh (Sham treatment); n = 16, WT-MI (Infarction treatment); n = 12, GPR176 KO-MI (Infarction treatment); n = 9. Error bars represent the mean ± SEM. * P <0.05, ** P <0.01, *** P <0.001, unpaired two-sided student's t-test. Figure 5B: Graphs showing the weight ratio of the heart to the body weight and the weight ratio of the lungs to the body weight in the WT mice and the GPR176-KO mice 28 days after the myocardial infarction treatment. WT-Sh (Sham treatment); n = 18, KO-Sh (Sham treatment); n = 16, WT-MI (Infarction treatment); n = 12, GPR176 KO-MI (Infarction treatment); n = 9. Error bars represent the mean ± SEM. * P <0.05, ** P <0.01, *** P <0.001, unpaired two-sided student's t-test.

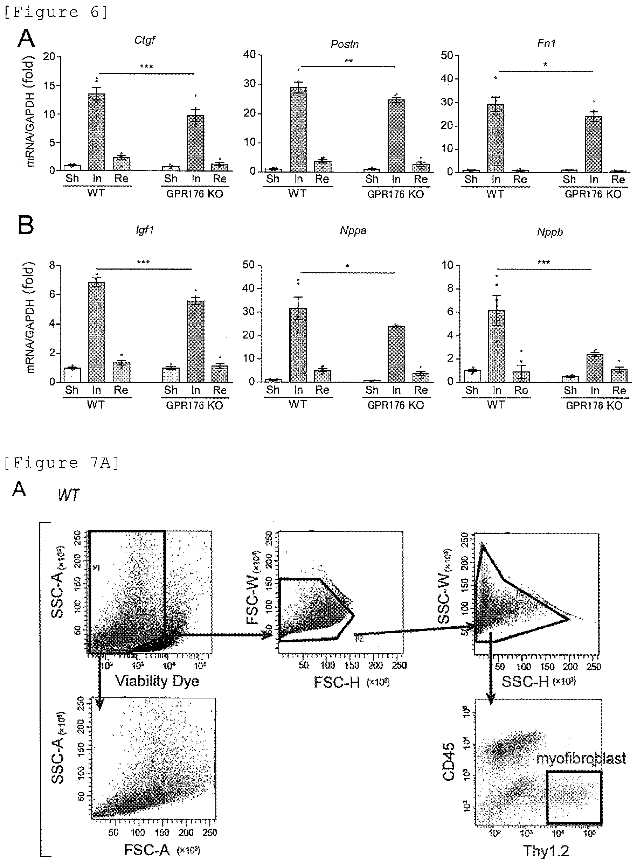

Figure 6 shows that the GPR176 deficiency reduces fibrosis after the myocardial infarction. Figures 6A and B: Graphs showing the mRNA expression levels of the fibrosis-related factors (Ctgf, Postn and Fn1) (A), and the cardiac hypertrophy genes (Igf1, Nppa and Nppb) (B) in the hearts of the WT mice and the GPR176-KO mice 7 days after the myocardial infarction. GAPDH was used as an internal control. WT-Sh (Sham treatment); n = 5, WT-In (Infarction treatment); n = 5, GPR176 KO-Sh (Sham treatment); n = 4, GPR176 KO Infarction treatment; n = 5. Error bars represent the mean ± SEM. "Sh" represents the sham-treatment group, "In" represents the infarcted region of the heart 7 days after the myocardial infarction treatment, and "Re" represents the region away from the infarcted region of the heart 7 days after the myocardial infarction treatment. * P <0.05, ** P <0.01, *** P <0.001, unpaired two-sided student's t-test.
Figure 7A is a graph showing the sorting of the cell fraction of the CD45(-)Thy1.2(+) from the WT mice. In the figure, "WT" and "myofibroblast" represent the results of the wild-type mice, and the cell population containing myofibroblasts, respectively. "Viability Dye" is a dye for determining dead cells.

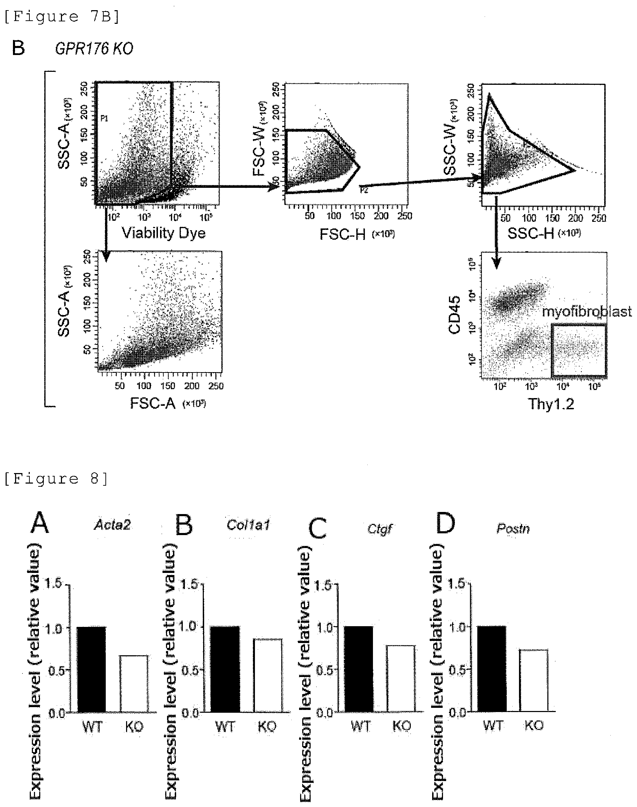

Figure 7B is a graph showing the sorting of the cell fraction of CD45(-)Thy1.2(+) from the GPR176 knockout mice. In the figure, "GPR176 KO" and "myofibroblast" represent the results of the GPR176 knockout mice, and the cell population containing myofibroblasts, respectively. "Viability Dye" is a dye for determining dead cells.
Figure 8 is graphs showing the measurement results of the expression levels of the fibrosis-related factors in each cell fraction. Figure 8A, B, C, and D show the measurement results of the expression levels of the Acta2 gene, the Colla1 gene, the Ctgf gene, and the Postn gene, respectively. In the figure, "WT" represents the result of the wild-type mice, and "KO" represents the result of the GPR176 knockout mice. The expression level of each gene is shown as a relative value to the expression level of the GAPDH gene.

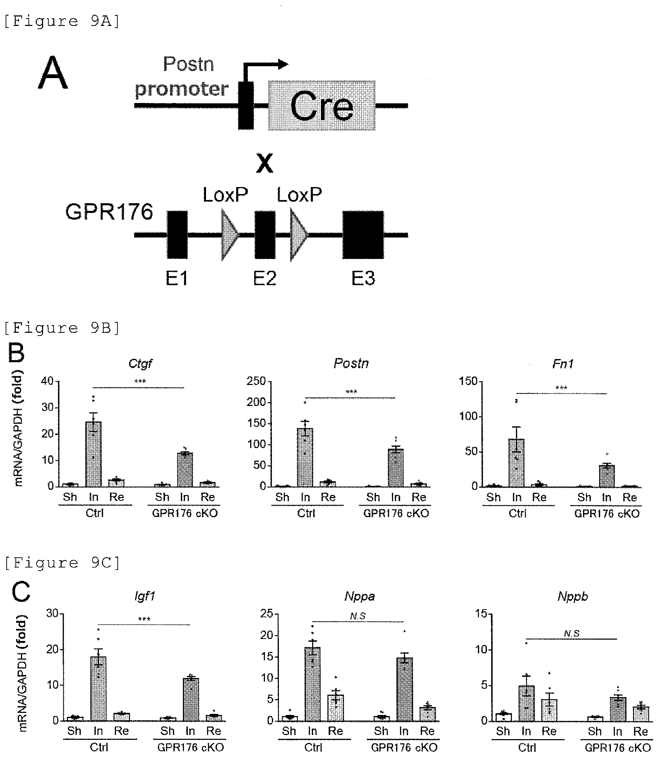

Figure 9A shows a schematic diagram of the myofibroblast-specific GPR176-deficient mice as used. The mice expressing Cre (DNA recombination enzyme) downstream the promoter of the periostin gene and the genetically modified mice in which the exon 2 of GPR176 was sandwiched between LoxP sequences (Cre recombinase target sequence) were crossbred to prepare the mice in which GPR176 was deficient specifically on the myofibroblasts.
Figure 9B is graphs showing the mRNA expression levels of the fibrosis-related factors (Ctgf, Postn and Fn1) in the hearts of the Ctrl (control) mice and the myofibroblast-specific GPR176-KO mice 7 days after the myocardial infarction treatment. "Sh", "In" and "Re" represent the sham-treatment group, the infarcted region of the hearts 7 days after the myocardial infarction treatment, and the region away from the infarcted region of the hearts 7 days after the myocardial infarction treatment, respectively. GAPDH was used as an internal control. Ctrl-Sh (Sham treatment); n = 8, Ctrl-In (Infarction treatment)); n = 6_{,} GPR176 cKO-Sh (Sham treatment); n = 7, GPR176 cKO-In (Infarction treatment)); n = 7. Error bars represent the mean ± SEM. * P <0.05, ** P <0.01, *** P <0.001, unpaired two-sided student's t-test.
Figure 9C is graphs showing the mRNA expression levels of the cardiac hypertrophy genes (Igf1, Nppa and Nppb) in the hearts of the Ctrl (control) mice and the myofibroblast-specific GPR176-KO mice 7 days after the myocardial infarction treatment. "Sh", "In" and "Re" represent the sham-treatment group, the infarcted region of the hearts 7 days after the myocardial infarction treatment, and the region away from the infarcted region of the hearts 7 days after the myocardial infarction treatment, respectively. GAPDH was used as an internal control. Ctrl-Sh(Sham treatment); n = 8, Ctrl-In(Infarction treatment)); n = 6, GPR176 cKO-Sh (Sham treatment); n = 7, GPR176 cKO-In (Infarction treatment)); n = 7. Error bars represent the mean ± SEM. * P <0.05, ** P <0.01, *** P <0.001, unpaired two-sided student's t-test.

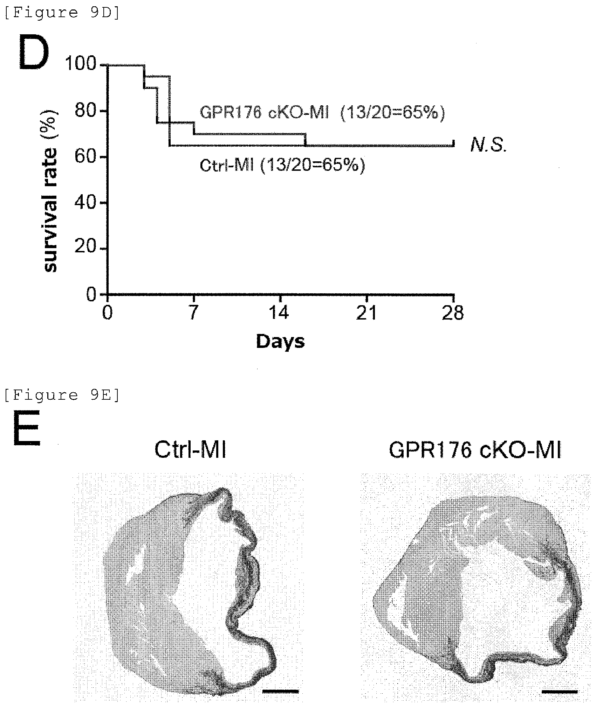

Figure 9D is a graph showing the Kaplan-Meier survival curve in the Ctrl mice and the GPR176 cKO mice 28 days after the infarction treatment. The difference between the Ctrl-MI and the GPR176 cKO-MI was evaluated by a log rank test. Ctrl; n = 20, GPR176 KO; n = 20.
Figure 9E is photographs showing the picrosirius red staining of the paraffin-embedded ventricular sections from the Ctrl mice and the GPR176 cKO mice 28 days after the infarction treatment. Scale bar, 1 mm.

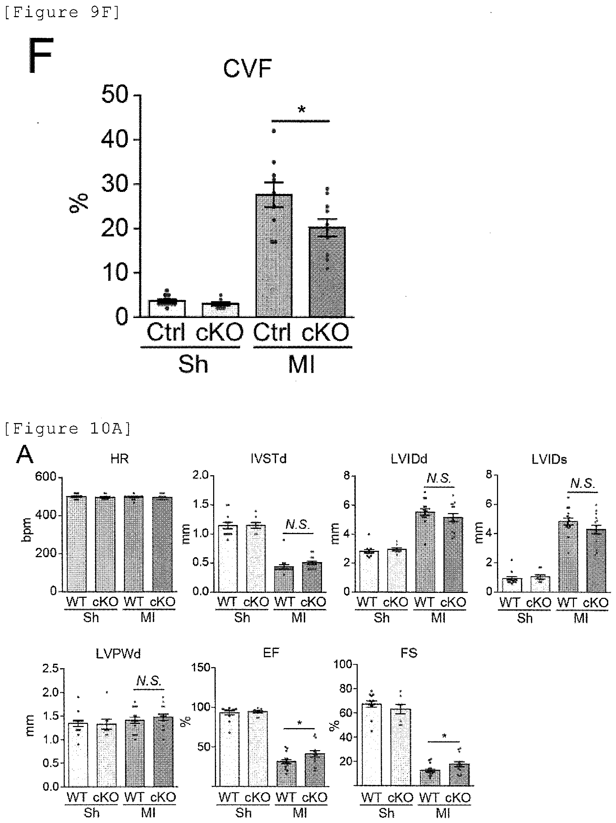

Figure 9F is a graph showing the quantitative data on the collagen volume fraction (CVF) of the Ctrl mice and the GPR176 cKO mice 28 days after the infarction treatment. Ctrl-Sh (Sham treatment); n = 12, GPR176 cKO-Sh (Sham treatment); n = 8, Ctrl-MI (Infarction treatment); n = 9, cKO-MI (Infarction treatment); n = 10. Error bars represent the mean ± SEM. * P <0.05, ** P <0.01, *** P <0.001, unpaired two-sided student's t-test.
Figure 10A is graphs showing the results of echocardiographic analysis of the hearts of the Ctrl mice and the GPR176 cKO mice 28 days after the infarction treatment. HR; Heart rate, IVSTd; Ventricular septal diastole, LVIDd; Left ventricular end diastole, LVID; Left ventricular end systole, LVPWd; Left ventricular posterior wall diastole, EF; Ejection fraction, FS; Fraction reduction. Ctrl-Sh(Sham treatment); n = 13, GPR176 cKO-Sh(Sham treatment); n = 8 Ctrl-MI(Infarction treatment); n = 15, GPR176 cKO-MI(Infarction treatment); n = 14. Error bars represent the mean ± SEM. * P <0.05, N.S. No significant difference, unpaired two-sided student's t-test.

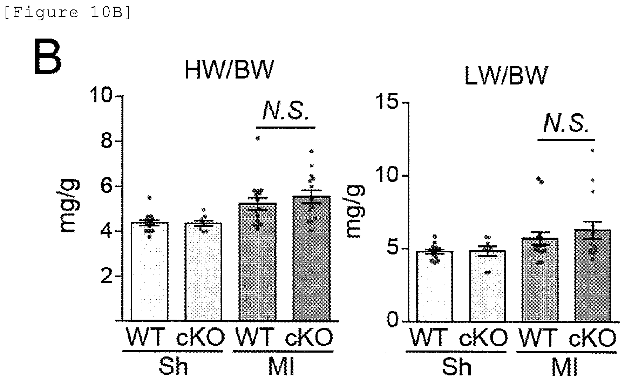

Figure 10B is graphs showing the ratio of the heart to the body weight and the ratio of the lungs to the body weight in the infarcted heart of the Ctrl mice and the GPR176 cKO mice 28 days after the infarction treatment. Ctrl-Sh(Sham treatment); n = 13, GPR176 cKO-Sh(Sham treatment); n = 8 Ctrl-MI(Infarction treatment); n = 15, GPR176 cKO-MI(Infarction treatment); n = 14ₒ Error bars represent the mean ± SEM. * P <0.05, N.S. No significant difference, unpaired two-sided student's t-test.

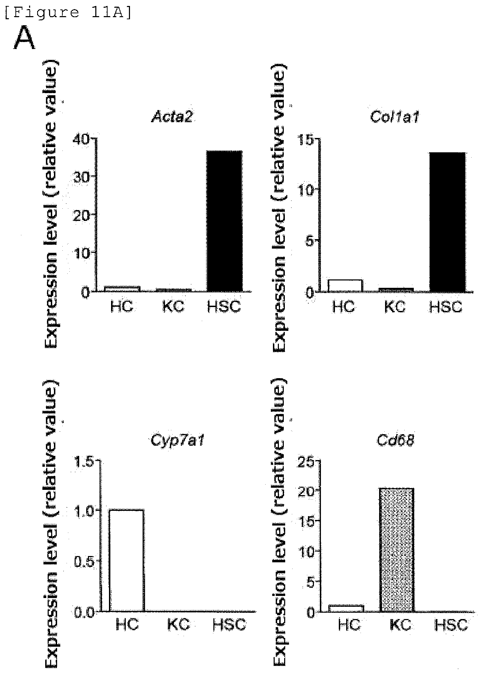

Figure 11A is graphs showing the measurement result of the expression level of a marker molecule for myofibroblast, a marker molecule for hepatocyte, and a marker molecule for macrophage in each cell fraction (HC: Hepatocyte, KC: Kupffer cell, HSC: Hepatic Stellate Cell) of the liver from the mice carrying the injured liver due to carbon tetrachloride administration. The expression levels of each gene of α-SMA (Acta2), Colla1 (Colla1), Cyp7a1 (Cyp7a1), and CD68 (Cd68) are shown as relative values to the expression level of the GAPDH gene.

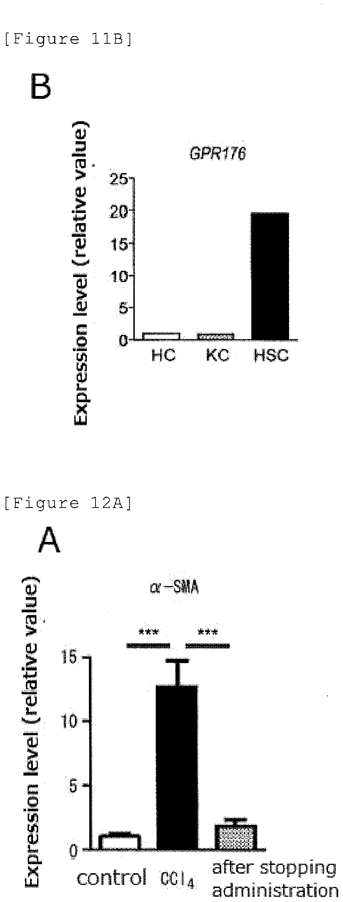

Figure 11B is a graph showing the measurement results of the expression level of GPR176 in each cell fraction (HC: Hepatocyte, KC: Kupffer cell, HSC: Hepatic Stellate Cell) of the liver from the mice carrying the injured liver due to carbon tetrachloride administration. The expression level is shown as a relative value to the expression level of the GAPDH gene.
Figure 12A is a graph showing the result of the quantified expression level of α-SMA by real-time RT-PCR in the livers of the mice of the control group not receiving carbon tetrachloride (n = 4-6), the group receiving carbon tetrachloride for 4 weeks (n = 6), and the group receiving carbon tetrachloride for 4 weeks, and then, with stopping the administration of carbon tetrachloride, continuing raising for 4 weeks (n = 5). In the figure, "control" represents the result of the control group, "CC14" represents the result of the group in which carbon tetrachloride was administered for 4 weeks, and "after stopping administration" represents the result of the group receiving carbon tetrachloride for 4 weeks, and then, with stopping the administration of carbon tetrachloride, continuing raising for 4 weeks. *** P <0.001, unpaired bilateral student's t-test.

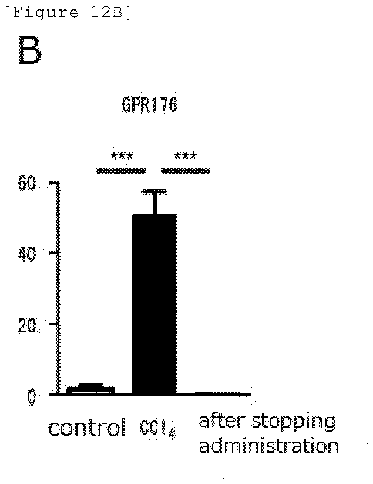

Figure 12B is a graph showing the result of the quantified expression level of GPR176 by real-time RT-PCR in the livers of the mice of the control group not receiving carbon tetrachloride (n = 4-6), the group receiving carbon tetrachloride for 4 weeks (n = 6), and the group receiving carbon tetrachloride for 4 weeks, and then, with stopping the administration of carbon tetrachloride, continuing raising for 4 weeks (n = 5). In the figure, "control" represents the result of the control group, "CC14" represents the result of the group in which carbon tetrachloride was administered for 4 weeks, and "after stopping administration" represents the result of the group receiving carbon tetrachloride for 4 weeks, and then, with stopping the administration of carbon tetrachloride, continuing raising for 4 weeks. The results of the group bred for 4 weeks are shown. *** P <0.001, unpaired bilateral student's t-test.

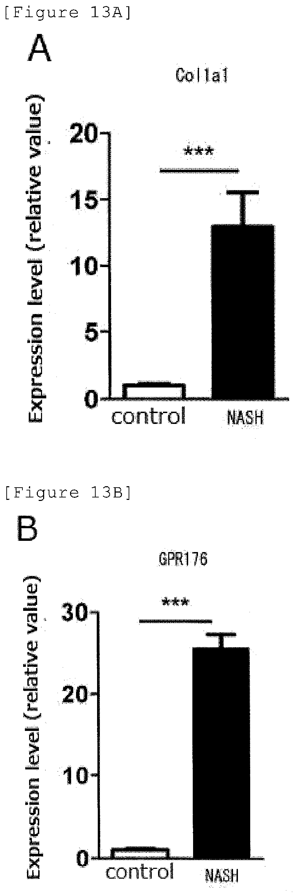

Figure 13A is a graph showing the results of the expression level of COlla1 quantified by real-time RT-PCR in the livers of the control mice (n = 5) receiving a normal diet and the NASH model mice (n = 5) receiving the diet for generating NASH. *** P <0.001, unpaired bilateral student's t-test.
Figure 13B is a graph showing the results of the expression level of GPR176 quantified by real-time RT-PCR in the livers of the control mice (n = 5) receiving a normal diet and the NASH model mice (n = 5) receiving the diet for generating NASH. *** P <0.001, unpaired bilateral student's t-test.

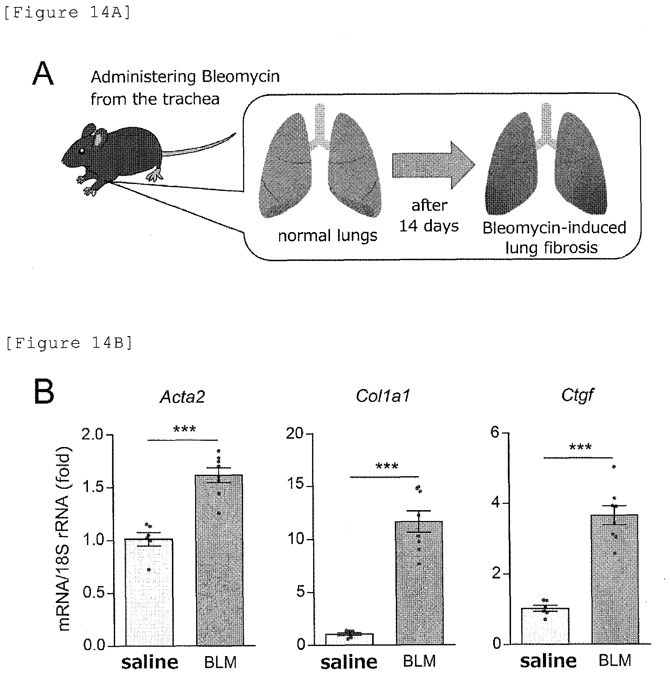

Figure 14A shows a schematic diagram for transtracheal administration of bleomycin to mice to induce the pulmonary fibrosis.
Figure 14B is graphs showing the mRNA expression levels of the fibrosis-related factors (Acta2, Ctgf and Postn) in the lungs of saline or BLM-treated mice. 18S rRNA was used as an internal control. Saline; n = 6, BLM; n = 8. Error bars represent the mean ± SEM. ** P <0.01, *** P <0.001, unpaired two-sided student's t-test.

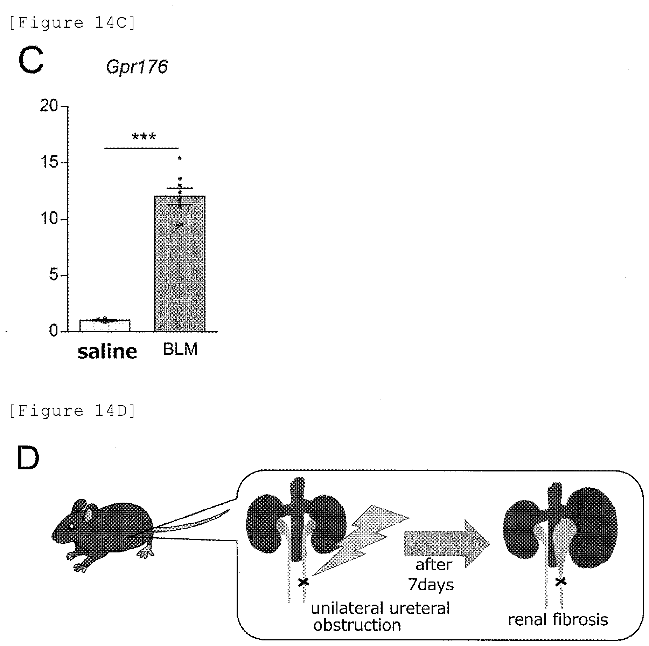

Figure 14C is a graph showing the mRNA expression levels of GPR176 in the lungs of the saline or BLM-treated mice. 18S rRNA was used as an internal control. Saline; n = 6, BLM; n = 8. Error bars represent the mean ± SEM. ** P <0.01, *** P <0.001, unpaired two-sided student's t-test.
Figure 14D is a schematic diagram for inducing renal fibrosis by unilateral ureteral obstruction (UUO) treatment of mice.

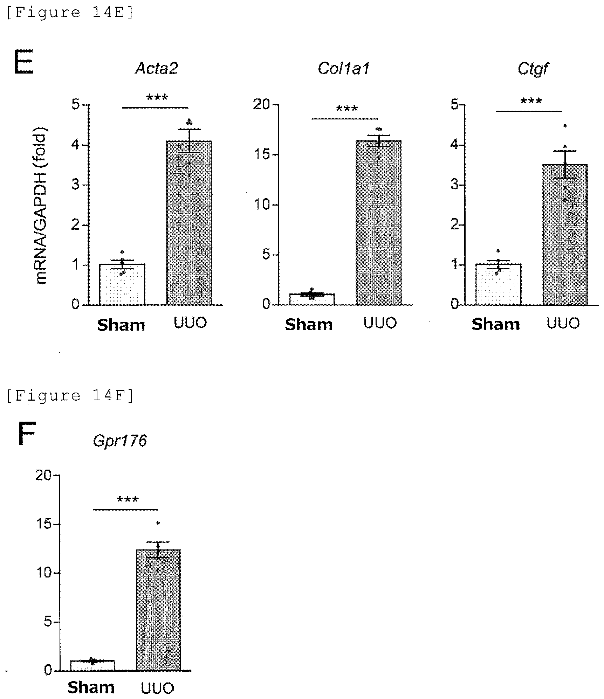

Figure 14E is graphs showing the mRNA expression levels of the renal fibrosis-related factors (Acta2, Colla1, Ctgf) in the sham-treated or UUO-treated mice. GAPDH was used as an internal control. Sham treatment; n = 5, UUO; n = 5. Error bars represent the mean ± SEM. ** P <0.01, *** P <0.001, unpaired two-sided student's t-test.
Figure 14F is a graph showing the mRNA expression levels of renal GPR176 in the sham-treated or UUO-treated mice. GAPDH was used as an internal control. Sham treatment; n = 5, UUO; n = 5. Error bars represent the mean ± SEM. ** P <0.01, *** P <0.001, unpaired two-sided student's t-test.

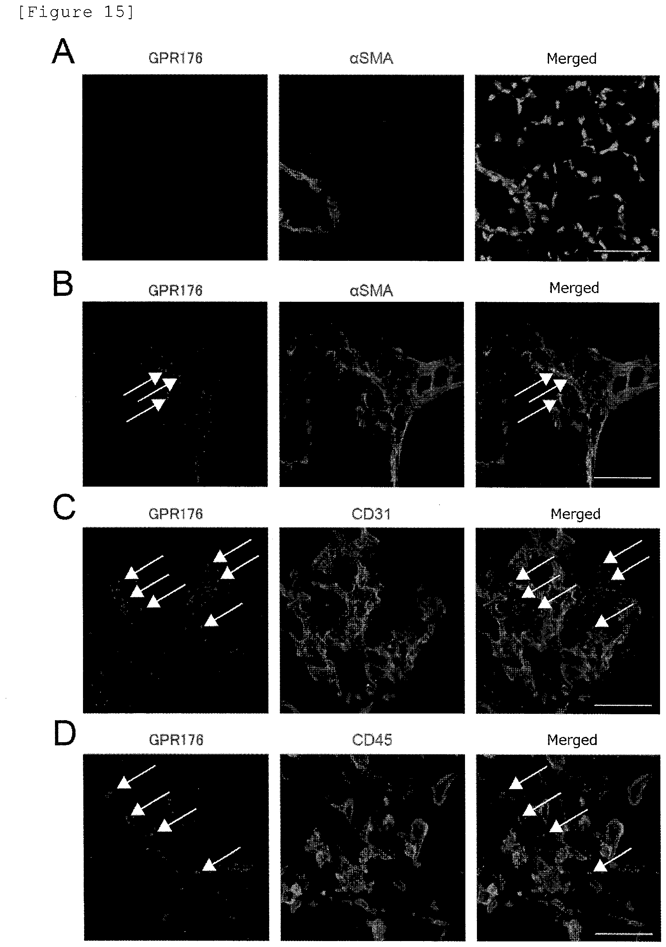

Figure 15 shows that GPR176 is also expressed on myofibroblasts during the fibrotic pathology in the lungs. Figure 15A: Photographs showing the results of the detected GPR176 mRNA by in situ hybridization (left) and the results of the immunostaining with the anti-αSMA antibody (center) in the lung sections of the Wild Type (WT) mice 7 days after BLM administration. "Merge" on the right is a superposition of them. Figure 15B, C, D: photographs showing the results of the detected GPR176 mRNA by in situ hybridization (left, respectively) and the results of the immunostaining with anti-αSMA (B), CD31 (C) and CD45 (D) antibodies (center of each) in the lung sections of the WT mice 7 days after BLM administration. "Merge" on the right is a superposition of them. Scale bar; 50 µm.

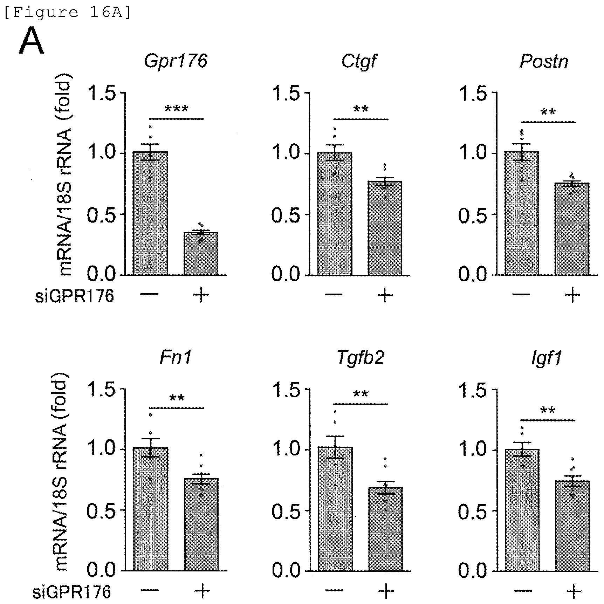

Figure 16A is graphs showing the mRNA expression levels of GPR176, the fibrosis-related factors (Ctgf, Postn, Fn1, and Tgfb2), and the cardiac hypertrophy gene (Igf1) in the cardiac myofibroblasts treated with the siRNA against GPR176. 18S rRNA was used as an internal control. n = 7-8ₒ Error bars represent the mean ± SEM. * P <0.05, ** P <0.01, *** P <0.001, unpaired two-sided student's t-test.

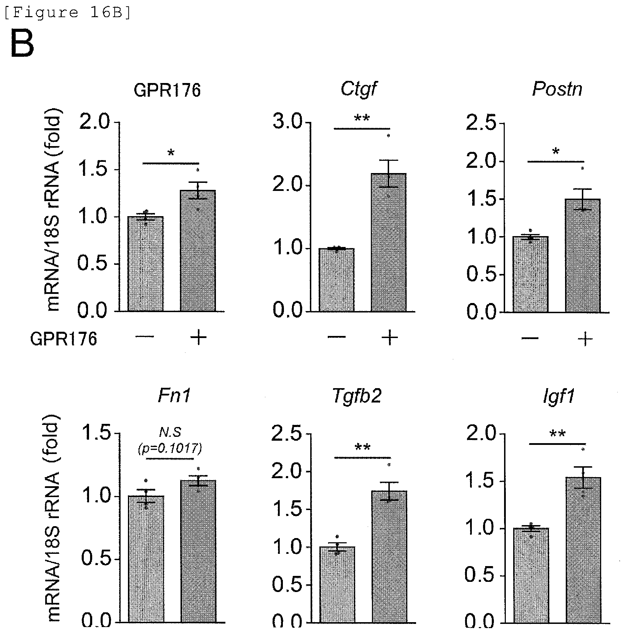

Figure 16B is graphs showing the mRNA expression levels of GPR176, the fibrosis-related factors (Ctgf, Postn, Fn1 and Tgfb2), and the hypertrophy gene (Igf1) in cardiac myofibroblasts overexpressing GPR176. 18S rRNA was used as an internal control. n = 4ₒ Error bars represent the mean ± SEM. * P <0.05, ** P <0.01, *** P <0.001, unpaired two-sided student's t-test.

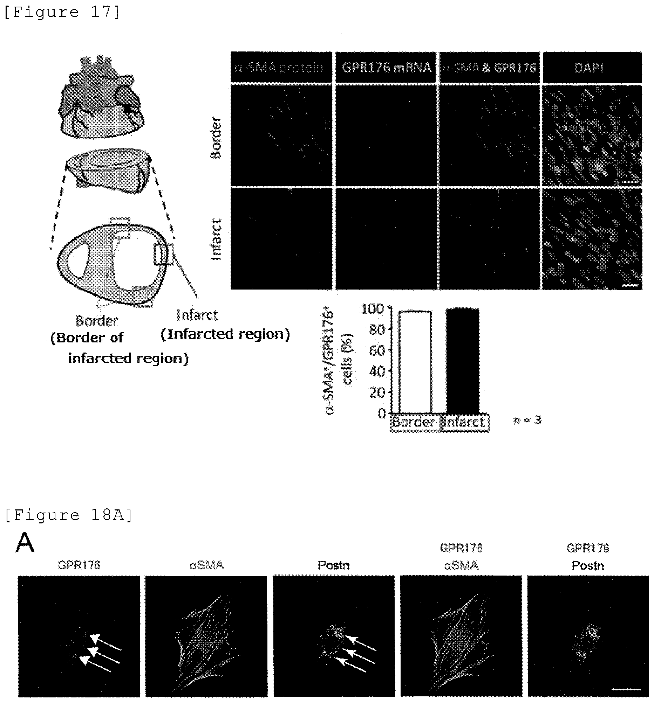

Figure 17 represents a photograph showing that the cells expressing the GPR176 mRNA express α-SMA in Border (border of infarcted region) or Infarct (infarcted region) of the mouse heart after the myocardial infarction treatment, and a graph in which the ratios of α-SMA expressed by the GPR176-positive cells were estimated in Border (border of infarcted region) or Infarct (infarcted region).
Figure 18A is photographs showing the results of the detection of the GPR176 mRNA and the Postn mRNA by in situ hybridization (left, center) and of the immunostaining with an anti-αSMA antibody in the myofibroblasts isolated from the mouse hearts 3 days after the infarction treatment.

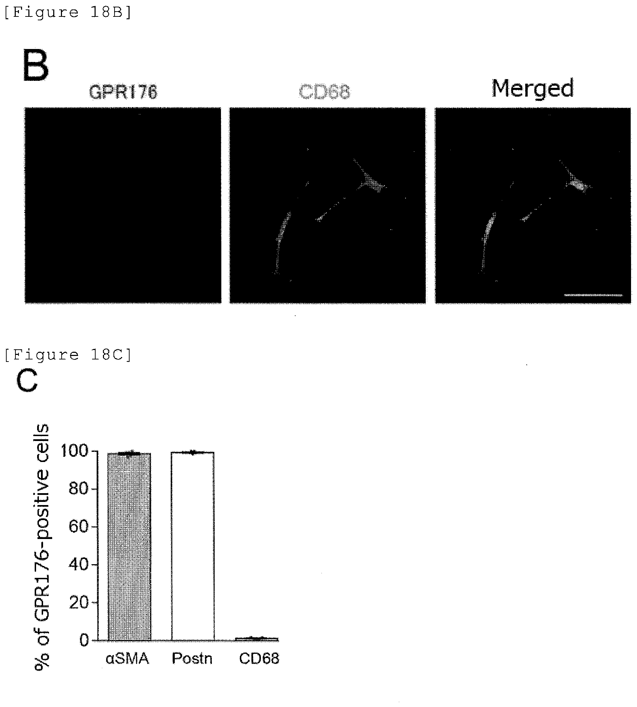

Figure 18B is photographs showing the results of the detection of the GPR176 mRNA by in situ hybridization (left), and of the immunostaining with an anti-CD68 antibody (center) in the leukocytes [CD45-positive cells] isolated from the mice 3 days after the infarction treatment. "Merge" on the right is a superposition of them.
Figure 18C is a graph in which the cells that were the GPR176 mRNA-positive cells were estimated among the markerpositive [αSMA-positive, Postn-positive, or CD68-positive] cells. The data were averaged from 5 hearts and, in each case, more than 100 cells were estimated.

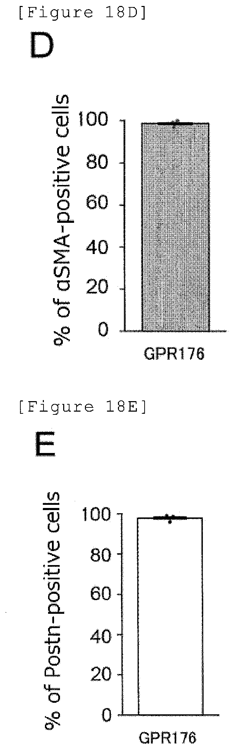

Figure 18D is a graph in which the cells that were the αSMApositive cells were estimated among the GPR176 mRNA-positive cells. The data were averaged from 5 hearts and, in each case, more than 100 cells were estimated.
Figure 18E is a graph in which the cells that were the Postn mRNA-positive cells were estimated among the GPR176 mRNA-positive cells. The data were averaged from 5 hearts and, in each case, more than 100 cells were estimated.

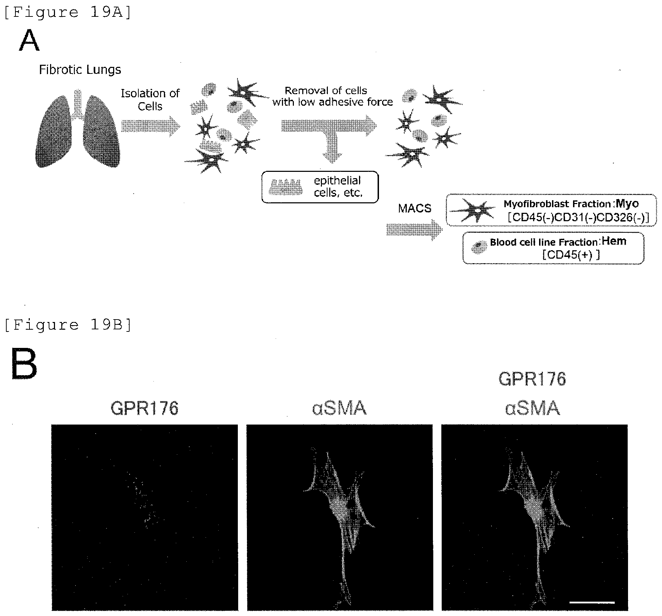

Figure 19A shows a methodology for isolating the myofibroblasts [CD45(-), CD31(-), CD326(-) cells] and the leukocytes [CD45(+) cells] from the WT mice on the 7th day after the BLM treatment by MACS.
Figure 19B shows photographs showing the results of the detection of the GPR176 mRNA by in situ hybridization, and of the immunostaining with an anti-CD68 antibody (center) in the myofibroblasts [CD45(-), CD31(-), CD326(-) cells] isolated from the mouse lung 7 days after the BLM treatment.

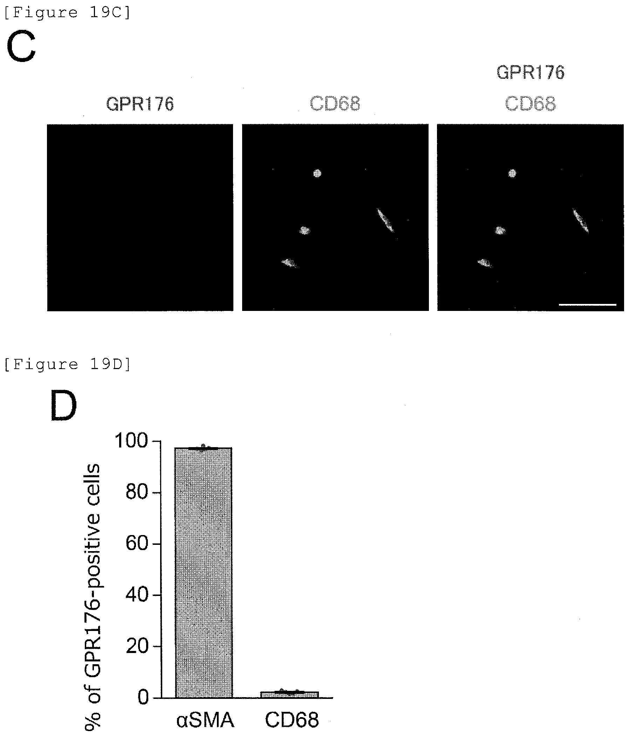

Figure 19C shows photographs showing the results of the detection of the GPR176 mRNA by in situ hybridization (left), and of the immunostaining with an anti-CD68 antibody (center) in the leukocytes [CD45(+) cells] isolated from the mouse lungs on 7 days after the BLM treatment.
Figure 19D is a graph in which the cells that were the GPR176 mRNA-positive cells were estimated among the αSMApositive or CD68-positive cells. The data were averaged from 5 hearts and, each case, more than 100 cells were estimated.

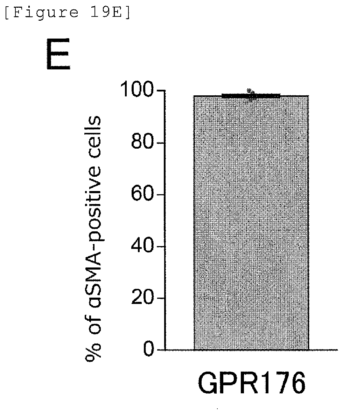

Figure 19E is a graph in which the cells that were the αSMApositive cells were estimated among the GPR176 mRNA-positive cells. The data were averaged from 5 hearts and each quantified more than 100 cells.

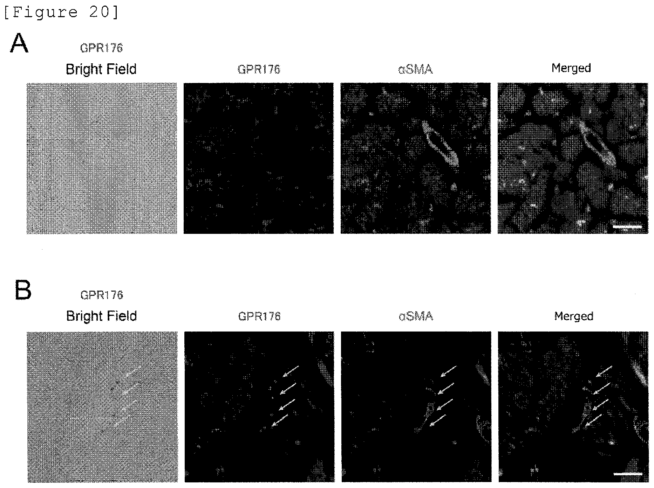

Figure 20 shows that GPR176 is also specifically expressed in the myofibroblasts in the human heart. Figure 20A: Photographs showing the result of the detection of the expression of the GPR176 mRNA by in situ hybridization (second from left: GPR176), and the result of the immunostaining with an anti-αSMA antibody (second from right: αSMA) in the cardiac sections of the patients not suffered from myocardial infarction. Bright Field is a photograph showing the result of the bright-field observation image. Figure 20B: Photographs showing the result of the detection of the expression of the GPR176 mRNA by in situ hybridization (second from left: GPR176), and the result of the immunostaining with an anti-αSMA antibody (second from right: αSMA) in the cardiac sections of the patients suffered from myocardial infarction. Arrows indicate the GPR176 mRNA. Scale bar; 20 µm

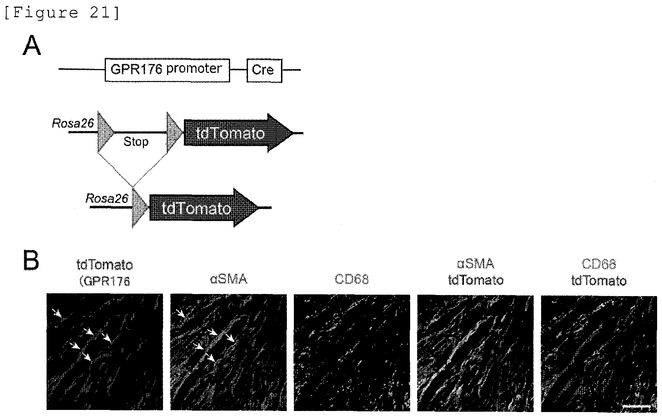

Figure 21 shows that most of the tdTomato-labeled cells in the heart of the GPR176 reporter mice after the myocardial infarction treatment are myofibroblasts. Figure 21A: Schematic diagram of the mouse lineage used. A genetically modified mouse in which Cre was knocked in the downstream of the GPR176 promoter was prepared, and this mouse was crossbred with a Rosa26-tdTomato mouse (Jackson Laboratory Stock No: 007914) to prepare a reporter mouse in which the GPR176-expressing cells were labeled with tdTomato. Figure 21B: Photographs showing images of GPR176 (far left) labeled by tdTomato, αSMA (second from left), and CD68 (center) stained on the infarcted cardiac sections.

### EMBODIMENTS FOR CARRYING OUT INVENTION

### <Preventive or treatment agents for fibrotic diseases>

In one embodiment, the present invention provides a pharmaceutical composition for preventing or treating fibrotic diseases, which comprises as an active ingredient an inhibitor of protein-coupled receptor 176 (GPR176), specifically an inhibitor of the GPR176 expression or an inhibitor of the GPR176 function. In the present invention, GPR176 includes human GPR176 and mouse GPR176, as well as variants thereof. The amino acid sequences and the nucleic acid sequences of variant 1, variant 2 and variant 3 of human GPR176 are set forth in SEQ ID NOs: 1 and 2 of the Sequence Listing for variant 1, SEQ ID NOs: 3 and 4 of the Sequence Listing for variant 2, and SEQ ID NOs: 5 and 6 of the Sequence Listing for variant 3, respectively. The amino acid sequences and the nucleic acid sequences of mouse GPR176 are set forth in SEQ ID NOs: 7 and 8 of the Sequence Listing, respectively. As described in the working examples hereinafter, GPR176 is a membrane surface marker molecule specific for myofibroblasts, which molecule promotes fibrosis. Therefore, an inhibitor of GPR176 can be used for the prevention or treatment of fibrotic diseases.

In the present invention, fibrosis means a phenomenon in which substances called extracellular matrix such as collagenous fibers (collagens) are increased in skin and internal organs, resulting in hardness of the skin and internal organs, and is also called "hardening". In the present invention, fibrotic diseases mean cardiac fibrosis, liver fibrosis, renal fibrosis, pulmonary fibrosis, systemic scleroderma, and skin sclerosis.

In the present invention, "inhibition" of "inhibitor" means a process, by inhibiting or suppressing the GPR176 expression or the GPR176 function, of (1) delaying the onset of the GPR176 activity; (2) slowing or stopping the progression, aggravation, or exacerbation of the symptoms of the GPR176 activity; (3) inducing remission of the symptoms of the GPR176 activity; or (4) facilitating the cure of the symptoms of the GPR176 activity.

In the present invention, examples of an inhibitor of the GPR176 expression include substances that inhibit the expression of the gene or nucleic acid represented by any one of (a) to (c) :
(a) the GPR176 gene set forth in any one of SEQ ID NOs: 2, 4 or 6 of the Sequence Listing,
(b) a nucleic acid that comprises a base sequence containing a deletion, a substitution or an addition of one or several bases in the GPR176 gene set forth in any one of SEQ ID NOs: 2, 4 or 6 of the Sequence Listing, and
(c) a nucleic acid that hybridizes under stringent conditions with a base sequence complementary to the GPR176 gene set forth in any one of SEQ ID NOs: 2, 4 or 6 of the Sequence Listing.

In the present invention, a gene or a nucleic acid as used in an embodiment in which the expression of a gene or a nucleic acid is inhibited (hereinafter, may be referred to as "target nucleic acid"), is a membrane surface marker molecule specific for myofibroblasts, which molecule may be any one of the GPR176 gene represented by the base sequence set forth in any one of SEQ ID NOs: 2, 4 or 6 discovered by the present inventors to promote fibrosis, or a nucleic acid that comprises a base sequence which contains a deletion, a substitution or an addition of one or several bases in the base sequence set forth in any one of SEQ ID NOs: 2, 4 or 6, or a nucleic acid that hybridizes under stringent conditions with a base sequence complementary to a nucleic acid comprising the base sequence set forth in any one of SEQ ID NOs: 2, 4 or 6, which nucleic acid has a base sequence encoding a protein represented by the amino acid sequence set forth in SEQ ID NO: 1 or a protein having the same function as the protein. Preferably, the gene or the nucleic acid is the GPR176 gene set forth in any one of SEQ ID NOs: 2, 4 or 6 of the Sequence Listing.

In the present invention, mRNA may be any mRNA encoded by the target nucleic acid or anyone encoding a protein encoded by the target nucleic acid. Preferably, mRNA is an mRNA encoded by the GPR176 gene.

The target nucleic acid of the present invention may be obtained as follows. First, the mRNA is extracted from human cells or tissues that produce the GPR176 protein, such as human myofibroblasts according to known techniques. The cells or tissues capable of producing the GPR176 protein may be identified by Northern blotting using a nucleic acid comprising a base sequence encoding the GPR176 protein or a part thereof, Western blotting using an antibody specific for the GPR176 protein, or the like. In the present invention, a guanidine/thiocyanate/hot phenol method, a guanidine/thiocyanate-guanidine/hydrochloric acid method or the like may be used as an extraction method, and a guanidine/thiocyanate cesium chloride method is preferable. In the present invention, the purification of mRNA may be carried out according to the conventional method, and include a method for purifying mRNA comprising adsorbing on an oligo (dT) cellulose column or the like, and eluting the mRNA extracted from human cells or tissues including human breast cancer tissues producing the GPR176 protein according to known techniques, or a method for fractionating mRNA by a sucrose density gradient centrifugation method, or the like. Alternatively, a commercially available extracted mRNA may be used without extracting the mRNA.

In one embodiment of the invention, the inhibitor of the GPR176 expression is preferably selected from the group consisting of an siRNA, an antisense and a ribozyme.

A ribozyme according to the present invention means an RNA molecule that specifically cleaves a different single-stranded RNA molecule by a mechanism similar to that of a DNA restriction endonuclease. A ribozyme that recognizes and cleaves a specific base sequence in an RNA single strand may be prepared by modifying appropriately the nucleic acid sequence of RNA according to known techniques (Science, 239, p. 1412-1416, 1988).

An siRNA according to the present invention means a doublestranded RNA that suppresses the expression of a target nucleic acid, and means "RNAi agent", "short-chain interfering RNA", "short-chain interfering nucleic acid", and "siNA", all of which are nucleic acid molecules capable of inhibiting or downregulating a gene expression or a viral replication through sequence-specific RNA interference (RNAi) or gene-silencing. It may be composed of an RNA alone, or a fusion of a DNA and RNA. For example, an siRNA comprising the RNA sequences set forth in SEQ ID NOs: 9 and 10 of the Sequence Listing can be exemplified. The siRNA may be prepared according to a conventional method from the target nucleic acid and the information obtained by searching NCBI database. In the design of an siRNA oligonucleotide, a sequence comprising a GC content as close to 50% as possible is selected from the coding region of the mRNA encoded by the target nucleic acid. Ideally, the GC content should be between 45% and 55%. The region of 50 to 100 nucleotides near the AUG start codon or 50 to 100 nucleotides near the termination codon is avoided, and the 19 nucleotides following AA (adenine/adenine) is selected followed by adding the dTdT (deoxythymine/deoxythymine) to the 3'end of this sense chain of 19 nucleotides as an overhang. An overhang may be selected from an RNA oligo or an RNA/DNA chimeric oligo. Thymine (TT) and uracil (UU) are usually used as a two bases overhang, but other overhangs may also be used. An effect of the RNAi may be affected by blunting the siRNA ends, overhanging only the 5'end, or changing the length of the overhang. In the base sequence of the nucleotide, a sequence in which three or more guanosine or cytosine are contiguous should not be selected, and it is necessary to confirm that they are not homologous to any other gene. The antisense strand is a complementary strand of sense 19 nucleotides, and is added to its 3'end with dTdT. Based on the base sequence designed in this way, the sense strand and the antisense strand are synthesized by a DNA/RNA synthesizer. The synthesized sense strand and antisense strand are purified by a NAP-10 column or the like, concentrated and dried, again dissolved in a buffer, heated, and annealed to provide a doublestranded siRNA. In addition to the method for preparing an siRNA using a DNA/RNA synthesizer, it is also possible to incorporate the siRNA target sequence of the present invention into an expression vector together with a loop sequence so as to express it intracellularly, leading to the suppression of the expression of the target nucleic acid. Also, it is possible to separately express the sense strand and the antisense strand of the siRNA target sequence of the present invention in a cell, and hybridize in the cell to provide the siRNA, leading to the suppression of the expression of the target nucleic acid. Further, it is also possible to introduce a long dsRNA containing the siRNA target sequence of the present invention into a cell and cleave it into the cell to provide the siRNA, leading to the suppression of the expression of the target nucleic acid.

The siRNA of the present invention can be used as a substance for regulating the expression of the target nucleic acid, as an inhibitor of fibrosis, and as a pharmaceutical composition for preventing or treating fibrotic diseases, since the siRNA inhibits the expression of the target nucleic acid (GPR176 gene). Examples of such siRNAs include an siRNA comprising the RNA sequences set forth in SEQ ID NOs: 9 and 10 of the Sequence Listing.

The nucleic acid comprising the antisense sequence according to the present invention is a nucleic acid comprising a base sequence that suppresses the expression of the target nucleic acid, and is prepared according to a conventional method. First, a target candidate site of the mRNA encoded by the target nucleic acid is selected. As a selection method, a method for predicting the higher-order structure of mRNA based on energy calculation (Methods in Enzymol., 180, p. 262, 1989; Ann. Rev. Biophys. Biophys. Chem., 17, p. 167, 1988), Random oligo/RNase H method (Nucleic Acid Res., 25, p. 5010, 1997), Reverse transcription enzyme method (Nature Biotech, 15, p. 537, 1997), Fluorescent nucleic acid probe method (Nucleic Acid Res., 27, p. 2387, 1999), FRET method (Biochemistry, 31, p. 12055, 1992) and the like may be used. Next, the sequence of the antisense oligonucleotide is determined from the candidate region of the selected mRNA. At this time, the antisense oligonucleotide is designed to have the chain length of 15 to 30-mer generally, not to form a double strand or a self-stem-loop structure from the antisense oligonucleotide itself, to avoid a sequence that line up four or more of continuous Gs since it is likely to interact with a protein, and to avoid a "CpG" sequence in the antisense oligonucleotide since it binds to a B cell receptor. The structure of the antisense oligonucleotide may be selected from a phosphate-bound type (a natural type, a phosphorothioate type, a methylphosphonate type, a phosphoroamidate type, a 2'-O-methyl type) and a non-phosphate type (a morpholidate type, and polyamide nucleic acid) and the like. It is also possible to introduce a peptide or cholesterol in order to enhance the cell permeability, or to introduce an alkylating agent or a photocrosslinking agent in order to impart a crosslinking performance. The antisense oligonucleotide designed in this way is prepared by a DNA synthesizer or the like, and purified by reverse phase HPLC, ion exchange HPLC, gel electrophoresis, ethanol precipitation or the like.

The nucleic acid comprising the antisense sequence of the present invention can be used as a substance for regulating the expression of the target nucleic acid, as an inhibitor of fibrosis, and as a pharmaceutical composition for preventing or treating fibrotic diseases, since the nucleic acid inhibits the expression of the target nucleic acid (GPR176 gene).

In one embodiment of the invention, the pharmaceutical composition of the present invention comprises an inhibitor of the GPR176 function. Inhibitors of the GPR176 function include a substance specifically binding to the GPR176 protein. In the present invention, the substances specifically binding to the GPR176 protein include those selected from the group consisting of an antibody, an antibody fragment and an aptamer.

A protein as used in an embodiment in which a fibrosis is inhibited by inhibiting the function of the protein encoded by the target nucleic acid (hereinafter referred to as "target protein"), is a protein having an amino acid sequence encoded by the target nucleic acid, which protein has the amino acid sequence set forth in SEQ ID NO.1, and a protein which contains a substitution, a deletion, or an insertion of 1 to 10, preferably 1 to 7, and more preferably 1 to 5 amino acids in said amino acid sequence, and which is involved in the growth mechanism of a cancer cell, or at least which presence is involved in the fibrosis mechanism like the GPR176 protein represented by the amino acid sequence set forth in SEQ ID NO: 1.

In the present invention, examples of an antibody include a polyclonal antibody, a monoclonal antibody, a chimeric antibody, and a humanized antibody. Examples of the antibody include a full-length antibody, a single-chain molecule, a bifunctional molecule, an scFv, a diabody, a single domain antibody (VHH), a chimeric antibody, and an immunoadhesion factor. Examples of an "Antibody fragment" include a fragment of Fv, Fv', Fab, Fab' and F(ab')₂. An "antibody" according to the present invention means a polypeptide containing one or more regions that bind to an epitope of an antigen of interest.

An "antibody fragment" may be obtained by treating an antibody with an enzyme such as a protease including papain, pepsin or the like (See Morimoto et al., J. Biochem. Biophys. Mehtods (1992) 24:107-17; Brennan et al., Science (1985) 229: 81). It may be also prepared by genetic recombination technique on the basis of the amino acid sequence of said antibody fragment.

A low molecular weight antibody having a modified structure of an "antibody fragment" may be constructed by utilizing an antibody fragment obtained by enzyme treatment or gene recombination. Alternatively, a gene encoding the entire low molecular weight antibody may be constructed, introduced into an expression vector, and then expressed in a suitable host cell (See e.g., Co et al., J. Immunol. (1994) 152: 2968-76; Better and Horwitz, Methods Enzymol. (1989) 178: 476-96; Pluckthun and Skerra, Methods Enzymol. (1989) 178: 497-515; Lamoyi, Methods Enzymol. (1986) 121: 652-63; Rousseaux et al., Methods Enzymol. (1986) 121: 663-9; Bird and Walker, Trends Biotechnol. (1991) 9: 132-7).

An "scFv" is a single-chain polypeptide in which two variable regions are linked via a linker or the like, if necessary. The two variable regions contained in the scFv are usually one heavy chain variable region (VH) and one light chain variable region (VL), but may be two VHs or two VLs. Generally, an scFv polypeptide contains a linker between the VH and VL regions, which forms the VH and VL pair portion required for antigen binding. Usually, in order to form a pair portion between a VH and a VL in the same molecule, a peptide linker having a length of 10 amino acids or more is generally selected as a linker linking the VH and the VL. According to the present invention, however, the scFV linker is not limited to such peptide linkers as far as it does not interfere with the formation of an scFv. Pluckthun, The Pharmacology of Monoclonal Antibody, Vol. 113 (Rosenburg and Moore ed., Springer Verlag, NY, pp. 269-315 (1994)) may be referred as the review of scFv.

In the present invention, an aptamer is a nucleic acid molecule or a peptide molecule that binds to a specific target molecule. An aptamer is usually prepared by selecting from a large pool of random sequences, but the native aptamers are also present in riboswitches. The aptamer can be used as a macromolecular drug for both basic research and clinical purposes. The Aptamer can be combined with a self-cleaving ribozyme in the presence of a target molecule therefor. More specifically, the aptamer may be classified as a nucleic acid aptamer such as a DNA or RNA aptamer, or a peptide aptamer. The former is usually composed of a strand (usually short) of oligonucleotide, while the latter is preferably composed of a short variable peptide domain attached to both ends of the protein scaffold. The nucleic acid aptamer is, in principle, a nucleic acid species that has been engineered through the repeated rounds of SELEX (systematic evolution of ligands by exponential enrichment) selected in vitro or equivalent so as to bind to various molecular targets, including a small molecule, a protein, a nucleic acid, and even a cell, a tissue and even an organism itself. The peptide aptamer is usually a peptide or protein designed to interfere with a different protein interaction within a cell. They are composed of a variable peptide loop attached to both ends of the protein scaffold. This bistructural constraint substantially increases the binding affinity of the peptide aptamer to a level that is comparable to that of an antibody (nanomolar range). The variable peptide loop typically contains 10 to 20 amino acids, and the scaffold may be any protein with a good solubility property. Currently, the bacterial protein thioredoxin-A is the most commonly used scaffold protein having the variable peptide loop inserted within the oxidation-reduction active site, which is the -Cys-Gly-Pro-Cys- loop in the wild-type protein, wherein the side chains of two cysteine residues can form a disulfide bridge. Selection of a peptide aptamer can be performed using different systems, and the system most widely used is currently the yeast two-hybrid system.

Aptamers provide any utility for bioengineering and therapeutic applications because they provide molecular recognition properties comparable to commonly used biomolecules, especially antibodies. In addition to their distinctive recognition, aptamers allow them to be fully manipulated in vitro, are easily produced by chemical synthesis, have a desirable storage property, and does not induce almost or completely any immunogenicity in therapeutic applications, thereby offering advantages over antibodies. As a result of the aptamers having inherently a low molecular weight, unmodified aptamers rapidly disappear from the bloodstream with a half-life of minutes to hours, primarily due to nuclease degradation and clearance from the body by the kidneys. The use of the unmodified aptamers is currently focused on the treatment in transient conditions such as blood coagulation, or the treatment of organs such as an eye wherein any localized delivery can be performed. This rapid clearance is advantageous in applications such as in vivo diagnostic imaging. Some modifications such as 2'-fluorinated pyrimidine binding, polyethylene glycol (PEG) binding, and a fusion with albumin or other half-life prolonging protein, may be utilized to increase the half-life of aptamers by days or even weeks.

Briefly, the inhibitor of GPR176 may be, for example, a substance that suppresses the expression of GPR176 at the mRNA level or the protein level, an antagonist against GPR176, or the like, or may be a substance that inhibits the activity of GPR176.

Examples of the antagonist against GPR176 include a substance that does not activate GPR176, a substance that inhibits the binding of the ligand to GPR176 among the substances specifically binding to GPR176.

Examples of the substances specifically binding to GPR176 include an antibody, an antibody fragment, an aptamer and the like. Examples of the antibody fragment include Fv, Fab, scFv and the like. The above antibody or the antibody fragment may be polyclonal or monoclonal. Moreover, examples of the aptamer include a nucleic acid aptamer, a peptide aptamer and the like.

Applications of antibodies, antibody fragments, and aptamers that are the substances specifically binding to GPR176 may be listed below. Antibodies to which a drug is added, or GPR176-neutralizing antibodies can be applied to the treatment of fibrosis. Antibodies with a fluorescent label or a radiation label can be applied to the detection and diagnosis of myofibroblasts and fibrotic sites. Further, an antibody-drug conjugate technology can be applied to specifically kill a myofibroblast that could express GPR176 and cause fibrosis.

As used herein, "treatment" means a method or process aimed at (1) delaying the onset of fibrotic diseases or a fibrotic state; (2) slowing or stopping the progression, aggravation or exacerbation of the symptoms of fibrotic diseases or a fibrotic state; (3) inducing remission of the symptoms of fibrotic diseases or a fibrotic state; or (4) facilitating the cure of fibrotic diseases or a fibrotic state. Treatment may be given as a prophylactic measure before the onset of the disease or the condition, or treatment may be given after the onset of the disease.

As used herein, "prevention" means a prophylactic action for the development of fibrotic diseases or a fibrotic state.

In the present invention, a pharmaceutical composition usually means a medicament for treating or preventing a disease, or for testing/diagnosis for a disease.

The pharmaceutical composition of the present invention can be formulated according to techniques well known to those skilled in the art. For example, it may be used parenterally in an injection form of a sterile solution or a suspension with a water or another pharmaceutically acceptable liquid. For example, it is believed to formulate the composition by appropriately combining it with a pharmacologically acceptable carrier or vehicle, specifically a sterile water or a saline, a vegetable oil, an emulsifier, a suspending agent, a surfactant, a stabilizer, a flavoring agent, an excipient, a vehicle, a preservative, a binder or the like, and mixing the same to form a unit dosage form required for generally accepted pharmaceutical practice. The amount of an active ingredient in these formulations is set so as to obtain an appropriate volume in the specified range.

Sterile compositions for injection may be prepared according to conventional formulations using vehicles such as a distilled water for injection.

Aqueous solutions for injection include, for example, a saline, an isotonic solution containing glucose and other adjuvants (e.g., D-sorbitol, D-mannose, D-mannitol, sodium chloride). Appropriate solubilizing agents such as an alcohol (ethanol, etc.), a polyalcohol (propylene glycol, polyethylene glycol, etc.), and a nonionic surfactant (polysorbate 80 (TM), HCO-50, etc.) may be used together.

Examples of an oily liquid include a sesame oil and a soybean oil, and benzyl benzoate and/or benzyl alcohol may be used together as a solubilizing agent. It may also be blended with a buffer (e.g., a phosphate buffer and a sodium acetate buffer), a soothing agent (e.g., procaine hydrochloride), a stabilizer (e.g., benzyl alcohol and phenol), an antioxidant. The prepared injection solutions are usually filled in a suitable ampoule.

The pharmaceutical composition of the present invention is preferably administered by parenteral route. For example, the composition may be an injection dosage form, a transnasal administration dosage form, a pulmonary administration dosage form, or a transdermal administration dosage form. For example, the composition may be administered systemically or locally by intravenous injection, intramuscular injection, intraperitoneal injection, subcutaneous injection, or the like.

The administration route may be appropriately selected depending on the age and symptoms of a patient. The dose of the pharmaceutical composition comprising the polypeptide may be set, for example, in the range of 0.0001 mg to 1000 mg per kg of body weight at one time. Alternatively, for example, the dose may be set in 0.001 to 100,000 mg per patient, but the present invention is not necessarily limited to these values. The dose and administration route vary depending on the weight, age, symptom, etc. of a patient, and those skilled in the art can set an appropriate dose and an administration route in consideration of these conditions.

In another aspect, the present invention is related to a method for preventing or treating fibrotic diseases, which comprises administering an effective amount of an inhibitor of protein-coupled receptor 176 (GPR176), specifically an inhibitor of the GPR176 expression or an inhibitor of the GPR176 function to a patient in need of such treatment or the like.

Further, in another embodiment, the present invention is related to an inhibitor of protein-coupled receptor 176 (GPR176) of the present invention, specifically an inhibitor of the GPR176 expression or an inhibitor of the GPR176 function for preventing or treating fibrotic diseases.

In yet another embodiment, the present invention is related to use of an inhibitor of protein-coupled receptor 176 (GPR176) of the present invention, specifically an inhibitor of the GPR176 expression or an inhibitor of the GPR176 function, for the preparation of a medicament for preventing or treating fibrotic diseases.

### <Nucleic acid molecule, etc.>

In another aspect, the present invention provides nucleic acid molecules selected from the group consisting of an siRNA, an antisense and a ribozyme, which inhibits the expression of:
(a) the GPR176 gene set forth in any one of SEQ ID NOs: 2, 4 or 6 of the Sequence Listing,
(b) a nucleic acid that comprises a base sequence containing a deletion, a substitution or an addition of one or several bases in the GPR176 gene set forth in any one of SEQ ID NOs: 2, 4 or 6 of the Sequence Listing, or
(c) a nucleic acid that hybridizes under stringent conditions with a base sequence complementary to the GPR176 gene set forth in any one of SEQ ID NOs: 2, 4 or 6 of the Sequence Listing. As described above, the nucleic acid molecule selected from the group consisting of an siRNA, an antisense and a ribozyme according to the present invention inhibits the expression of the target nucleic acid (GPR176 gene), and thus the molecule can be used as a substance for regulating the expression of the target nucleic acid, as an inhibitor of fibrosis, and as a pharmaceutical composition for preventing or treating fibrotic diseases.

Preferably, the nucleic acid molecule of the invention is introduced into a vector for gene therapy. Gene therapy often refers to as a treatment for a genetic disorder, but according to the present invention, gene therapy means a treatment for preventing or treating fibrotic diseases or suppressing the progression of fibrotic diseases. In the present invention, gene therapy may include in vivo copy insertion of a gene into a cell of a patient suffering from fibrotic diseases. Gene therapy may also include stopping a gene. Genetic recombination may also be used in ex vivo gene therapy. For example, a human stem cell, an immune cell or a cancer cell may be genetically modified for a variety of uses. Cells are modified to induce a differentiation, a trans-differentiation or a reprogramming. Cells may also be modified to serve as a vehicle that deliver a protein for therapeutics.

The present invention further provides a cell comprising the vectors according to the present invention. The cell according to the present invention can be used for cell therapy for preventing or treating fibrotic diseases, or suppressing the progression of fibrotic diseases.

### <Screening method>

In another embodiment, the present invention provides a method of screening for an inhibitor of GPR176, comprising confirming that the expression of a gene or a nucleic acid is inhibited, which is characterized by the use of the gene or the nucleic acid represented by any one of (a) to (c):
(a) the GPR176 gene set forth in any one of SEQ ID NOs: 2, 4, 6 or 8 of the Sequence Listing,
(b) a nucleic acid that comprises a base sequence containing a deletion, a substitution or an addition of one or several bases in the GPR176 gene set forth in any one of SEQ ID NOs: 2, 4, 6 or 8 of the Sequence Listing, or
(c) a nucleic acid that hybridizes under stringent conditions with a base sequence complementary to the GPR176 gene set forth in any one of SEQ ID NOs: 2, 4, 6 or 8 of the Sequence Listing, or
   a cell into which it has been introduced.

SEQ ID NOs: 2, 4 and 6 of the Sequence Listing show the base sequences of the human GPR176 gene, while SEQ ID NO: 8 shows the base sequence of the mouse GPR gene. In the method of screening for an inhibitor of GPR176 according to the present invention, both human GPR176 gene and mouse GPR176 gene can be used. Preferably, the human GPR176 gene is used.

A eukaryotic and prokaryotic host cell may be transformed by a suitable vector into which the gene or the nucleic acid isolated is incorporated. Further, an appropriate promoter and a sequence involved in the transformation may be introduced into such vector to express the mRNA or the protein encoded by the target nucleic acid in each host cell. As a vector in the present invention, a plasmid or a phage vector such as a lambda system, into which is inserted with a target nucleic acid, may be used. The plasmid may be either a self-replicating plasmid containing a transcriptional promoter region, or a plasmid that integrates into the chromosome of an animal cell. As an expression vector for a vertebrate animal cell that may be used in the present invention, a vector comprising a promoter normally located upstream of a gene to be expressed, an RNA splice site, a polyadenylation site, a transcription termination sequence, and the like, may be used, and the vector may contain a replication origin, if necessary. Examples of the expression vector include, but are not limited to, pSV2dhfr with the initial promoter of SV40 (Mol. Cell. Biol., 1, p.854-864, 1981), pEF-BOS with the human prolongation factor promoter (Nucleic Acids Res., 18, p.5322, 1990), pCEP4 with cytomegalovirus promoter (Invitrogen), and the like. For example, eukaryotic host cells include cells such as a vertebrate, an insect, and a yeast, and examples of the vertebrate cell include, but are not limited to, COS cells of monkey cells (Cell, 23, p.175-182, 1981), a dihydrofolate reductase-deficient strain of Chinese hamster ovary cells (CHO) (Proc. Natl. Acad. Sci. USA, 77, p.4216-4220, 1980), HEK293 cells derived from human fetal kidneys and 293-EBNA cells into which the EBNA-1 gene of Epstein-Barr virus has been introduced (Invitrogen), and the like.

Confirmation if the expression of a gene or a nucleic acid is inhibited can be typically performed using real-time Polymerase Chain Reaction (PCR), DNA microarray methods.

More specifically, the method of screening according to the present invention is related to a method of screening for an inhibitor of GPR176, which comprises;
(1) preparing a candidate compound,
(2) contacting said candidate compound to a cell comprising the gene or nucleic acid represented by any one of (a) to (c):
   (a) the GPR176 gene set forth in any one of SEQ ID NOs: 2, 4, 6 or 8 of the Sequence Listing,
   (b) a nucleic acid that comprises a base sequence containing a deletion, a substitution or an addition of one or several bases in the GPR176 gene set forth in any one of SEQ ID NOs: 2, 4, 6 or 8 of the Sequence Listing, or
   (c) a nucleic acid that hybridizes under stringent conditions with a base sequence complementary to the GPR176 gene set forth in any one of SEQ ID NOs: 2, 4, 6 or 8 of the Sequence Listing, and
(3) determining whether or not said candidate compound suppresses the expression of said gene or nucleic acid.

Various methods as shown below may be used for selecting a strain transfected with the target nucleic acid from the cells obtained as described above. Specifically, a method of directly confirming the presence of nucleic acid, a method of selecting as a strain expressing mRNA, and the like are used.

In step (3), for example, the method described in Foster et al., 2019, Cell 179, 89 5-908 may be used for "determining whether or not said candidate compound suppresses the expression of said gene or nucleic acid".

### Method of screening by use of a synthetic oligonucleotide probe

An oligonucleotide corresponding to the target nucleic acid is synthesized. In this case, either a nucleotide sequence derived using the frequency of codon usage or a plurality of nucleotide sequences in which possible nucleotide sequences are combined may be used, and in the latter case, inosine may be included to reduce the number of the nucleotide sequences. The resultant sequence is used as a probe (which is labeled with 32P or 33P) to hybridize the DNA of the transformant denatured and fixed on nitrocellulose filter, and the obtained positive strain is searched and selected.

### Method of screening by use of a probe prepared by Polymerase Chain Reaction (PCR)

Oligonucleotides of the sense primer and antisense primer corresponding to a part of the target nucleic acid are synthesized, and these are combined to perform PCR (Science, 239, p. 487-491, 1988), thereby amplifying the target nucleic acid. As a template DNA used therein, a cDNA synthesized by a reverse transcription reaction from the mRNA from a cell producing a target molecule for drug discovery or a genomic DNA can be used. The DNA fragment thus prepared is labeled with 32P or 33P, and colony hybridization or plaque hybridization in which the resultant fragment is used as a probe is performed to select a strain comprising the target nucleic acid of interest.

In yet another embodiment, the present invention provides a method of screening for a substance that inhibits the function of the GPR176 protein, characterized by the use of: the GPR176 protein having the amino acid sequence set forth in any one of SEQ ID NOs: 1, 3, 5 or 7 of the Sequence Listing, or a variant GPR176 protein which contains a substitution, a deletion or an additions of one or several amino acid residues in the GPR176 protein, and which has the same activity as said GPR176 protein, and more specifically, a method of screening for a substance that inhibits the function of the GPR176 protein, which comprises;
(1) preparing a candidate compound,
(2) contacting said candidate compound to the GPR176 protein having the amino acid sequence set forth in any one of SEQ ID NOs: 1, 3, 5 or 7 of the Sequence Listing, or a variant GPR176 protein which contains a substitution, a deletion or an addition of one or several amino acid residues in the GPR176 protein, and which has the same activity as said GPR176 protein, or to a cell expressing said GPR176 protein or said variant GPR176 protein, and
(3) determining whether or not said candidate compound inhibits the function of said GPR176 protein or said variant GPR176 protein.

SEQ ID NOs: 1, 3 and 5 of the Sequence Listing show the amino acid sequences of the human GPR176 protein, while SEQ ID NO: 7 shows the amino acid sequence of the mouse GPR protein. In the method of screening for an inhibitor of GPR176 according to the present invention, both human GPR176 protein and mouse GPR176 protein can be used. Preferably, the human GPR176 protein is used.

When the target protein is fused with a marker protein in frame and the fusion product is expressed, it is possible to confirm the expression of the target protein, confirm the intracellular localization of the target protein, perform the purification of the target protein, and the like. Examples of the marker protein include FLAG epitope, Hexa-Histidine tag, hemagglutinin tag, myc epitope and the like. Also, when an amino acid sequence specifically recognized by proteases such as an enterokinase, factor Xa, thrombin and the like is inserted between the amino acid sequences of the marker protein and the target protein, the marker protein portion may be cleaved and removed by these proteases. For example, there is a report in which a muscarinic acetylcholine receptor and a hexahistidine tag are linked through a thrombin recognition sequence (J. Biochem., 120, p. 1232-1238, 1996). A cell transformed with the vector into which a fusion product wherein a base sequence encoding these marker proteins and a target nucleic acid are fused in frame is incorporated, may be used to screen for a substance that regulates the function of a target nucleic acid or a target protein.

More specifically, according to the method of screening of the present invention, a method of screening for a prophylactic or therapeutic agent for fibrotic diseases, which comprises a step of culturing myofibroblasts in the presence of a candidate compound, a step of quantifying the expression level of the mRNA of the GPR176 gene or the GPR176 protein in the cultured myofibroblasts, and a step of judging the candidate compound to be a prophylactic or therapeutic agent for fibrotic diseases when the quantified expression level of the mRNA of said GPR176 gene or said GPR176the protein is decreased as compared with that of the control.

For a candidate compound, for example, a compound library or the like may be used. Examples of a control include the myofibroblasts cultured in the absence of any candidate compound and the like. The mRNA of the GPR176 gene may be quantified by, for example, microarray analysis or real-time RT-PCR. Further, the expression level of the GPR176 protein may be quantified by, for example, analysis using a protein chip or Western blotting.

### <Biomarkers, etc.>

According to another aspect, the present invention provides a method of determining the severity in fibrotic diseases, which comprises;
(a10) a step of measuring the amount of GPR176 on myofibroblasts of a subject (test biomarker amount),
(b10) a step of comparing the amount of the test biomarker with the amount of GPR176 on myofibroblasts of a healthy subject (control biomarker amount), and
(c10) a step of determining that fibrotic diseases become serious when the test biomarker amount is larger than the control biomarker amount.

In this regard, the present invention provides, in yet another aspect, a biomarker that is GPR176 of myofibroblasts which enable to determine the severity in fibrotic diseases. Further, the present invention provides the use of GPR176 as a biomarker which enable to determine the severity in fibrotic diseases.

The present inventors have found that GPR176 is a myofibroblast-specific membrane surface marker molecule, which is a molecule that promotes a fibrosis, and which is associated with fibrotic diseases, especially severe fibrotic diseases. The method, the biomarker, and GPR176 according to the present invention may be used to determine the severity of a subject in advance, and therefore it becomes possible to predict in advance if a patient is suffering from a severe fibrotic disease, which is advantageous for the prevention of fibrotic diseases.

The amount of GPR176 on myofibroblasts may be measured by immunological methods if there is an antibody against GPR176. For example, it can be measured by an ELISA method well known to those skilled in the art. Detection of mRNA of GPR176 may be performed by, for example, an RNA scope method (Advanced Cell Diagnostics), which is a type of super-sensitive in situ hybridization. In the RNA scope method, if the mRNA of the target molecule is expressed, it can be detected in dots by using a probe specific for the molecule. The RNA may be also recovered from myofibroblasts, and measured by real-time RT-PCR.

The myofibroblasts of a subject are collected by biopsy, and the amount of GPR176 is measured.

### <Kits for detection of myofibroblasts>

In the embodiment, the present invention provides a kit for detecting myofibroblasts, which includes a primer set for amplifying the cDNA of GPR176, a probe that specifically hybridizes with the mRNA of GPR176, or a substance specifically binding to the GPR176 protein.

As described below in the examples, the inventors have demonstrated that GPR176 is little expressed in the heart, liver, and kidneys of a living body in a normal state, and the expression of GPR176 is significantly increased only when each organ is in a fibrotic state. The inventors also have demonstrated that the GPR176 expression is specific for myofibroblasts that execute a fibrosis. Therefore, GPR176 is a novel myofibroblast-specific marker protein.

In the kit according to the present embodiment, the primer set is not particularly limited as long as it can amplify the cDNA of the GPR176 gene of the animal species to be diagnosed. The probe that specifically hybridizes with the mRNA of GPR176 is not particularly limited as long as it specifically hybridizes with the mRNA of the GPR176 gene. The probe may be immobilized on a carrier to form a DNA microarray and the like. Further, the specifically binding substance is the same as that described above. The specifically binding substance may be immobilized on a carrier to form a protein chip and the like.

Conventionally, proteins such as α-SMA, periostin, and the like have been used as a marker for myofibroblasts, which are cells executing a fibrosis. However, these are all intracellular proteins. In other words, conventionally, any cell membrane protein specifically expressed on myofibroblasts has not been known.

On the other hand, as described below in the examples, GPR176 is a cell membrane protein that is not expressed in the originating cell, but whose expression is increased when the cell differentiates into myofibroblasts. Since GPR176 is a cell membrane protein, it is possible to visualize the myofibroblasts invasively or non-invasively by labeling an antibody specific for GPR176 with a fluorescent or radioisotope, for example.

### <Other embodiments>

Since GPR176 is a cell membrane protein, an antibody specific for GPR176, for example, can be used to specifically deliver pharmaceutical agents to myofibroblasts.

All references cited in this specification, including publications, patent documents, etc., are individually and specifically incorporated by reference, and their entireties are specifically described by reference in this specification to the extent that they are incorporated herein.

Hereinafter, the present invention will be described in more detail by way of the working examples, but it should be noted that these would not limit the scope of the present invention and merely illustrate the invention.

### Examples

### Example 1

### Microarray analysis using myocardial infarction model mouse

Microarray analysis was performed to search for any cell membrane protein responsible for fibrosis. Specifically, microarray analysis was performed using the heart tissue removed from the myocardial infarction model mouse in which the left anterior descending artery of the left coronary artery of the mouse heart was ligated with a suture, and the heart tissue removed from a sham-treated mouse as a control, so as to search for any cell membrane protein whose expression level was increased significantly only in the myocardial infarction treatment group. In addition, any protein that were significantly expressed on myofibroblasts was selected. As a result, an orphan receptor whose function was unknown so far, GPR176, was found as a cell membrane protein responsible for fibrosis.

### Example 2

### Expression of GPR176 in myocardial infarction model mice

The myocardial infarction model mice were used to confirm the expression of GPR176. Specifically, the same myocardial infarction model mice (n = 5 to 6) same as in Example 1 were used to quantify the expression level of GPR176 in the heart tissue by real-time RT-PCR immediately (0 day), 3, 7 and 28 days after the myocardial infarction treatment. As a control, the mRNA expression level of GPR176 in the cardiac tissue of the sham-treated mice was quantified by real-time RT-PCR immediately (0 day), 3, 7 and 28 days after the treatment of the mice (n = 3 to 4).

Figure 1A is a graph showing the results of the mRNA expression level of GPR176 measured in the heart tissues of the myocardial infarction model mice and the control mice. As a result, it was shown that GPR176 was not expressed in the normal heart, whereas the expression level of GPR176 was increased significantly at the peak on the 7th day after the myocardial infarction treatment.

In addition, the sample same as above was used to measure the expression level of α-SMA, which is known as a marker for the myofibroblasts performing the organ fibrosis. Figure 1B is a graph showing the results of the expression level of α-SMA measured in the heart tissues of the myocardial infarction model mice and the control mice. As a result, it was shown that the transition in the expression level of α-SMA was very similar to the transition in the expression level of GPR176.

These results demonstrate that GPR176 should be responsible for fibrosis.

### Example 3

### Expression of GPR176 on myofibroblasts

We investigated which cells express GPR176.

Since there was no antibody against GPR176 that was available for immunostaining, a technique of RNA scope (Advanced Cell Diagnostics) was used to detect the GPR176 mRNA on the mouse heart sections. RNA scope is a type of an ultrasensitive in situ hybridization method, in which the expressed mRNA of the target molecule is detected in dots by use of a probe specific for that molecule (Wang, F. et al. J. Mol. Diagnostics 14, 22-29 (2012)). First, the expression of the GPR176 mRNA in the heart of the sham treatment group (sham) was examined, but no signal was detected (Figure 2A). This showed that GPR176 was not expressed on myocardial cells, resident fibroblasts, endothelial cells, etc. in the heat in steady-state conditions. On the other hand, on the 7th day after the infarction treatment when the expression level of GPR176 reached its peak, the expression of the GPR176 mRNA in the heart was examined and it was found that the many positive signals were detected in the tissue stroma (Right side of Figure 2A, Black arrows). Various leukocytes such as macrophages, neutrophils and T cells, as well as myofibroblasts differentiated from various cells, newly appear in the stroma of the heart after the myocardial infarction (Davis, J. & Molkentin, J. D., J. Mol. Cell. Cardiol. 70, 9-18 (2014); Liehn, E. A., Postea, O. , Curaj, A. & Marx, N., J. Am. Coll. Cardiol. 58, 2357-2362 (2011)). Therefore, GPR176 is expected to be expressed on leukocytes or myofibroblasts that exist in the stroma of the heart after the myocardial infarction.

Then, the cell fractions containing the leukocytes or the myofibroblasts were harvested from the mouse heart after the myocardial infarction by the use of the expression of CD45 (membrane surface marker molecule for leukocytes) or PDGFRα (membrane surface marker molecule for all fibroblasts including myofibroblasts) as an index, and the expression levels of GPR176 in these cell groups were compared. Specifically, the cells were isolated by digesting the heart with a collagenase solution on the third day after the infarction treatment, and then the cell fractions containing the leukocytes [CD45(+) PDGFR-α(-) cells] and the myofibroblasts [CD45(-) PDGFR-α(+) cells] were harvested using FACS AriaIII (Takeda, N. et al. J. Clin. Invest. 120, 254-265 (2010)) (Figure 2B) . First, the cells were gated with the dyes for dead cell determination and SSC-A to sort the viable cells, and then with FSC-H and FSC-W, and SSC-H and SSC-W to eliminate the doublet cells. The CD45(-) PDGFR-α(+) cells (fibroblasts including myofibroblasts) and the CD45(+) PDGFR-α(-) cells (leukocytes) were each fractionated, and then the RNAs were extracted from each of the cell fractions. Real-time RT-PCR method was used to examine whether or not the cell fractions recovered in this way were in high purity. When the expression of a marker for macrophages CD68 was examined, it was shown that CD68 (Cd68) was expressed only in the fraction of the leukocytes (Hem) (Figure 2C). On the other hand, the expression of a marker molecule for myofibroblasts α-SMA (Acta2) was high in the cell fraction containing myofibroblasts (Myo) [CD45(-) PDGFR-α(+) cells] (Figure 2C). The expression of Colla1 (Colla1) was also high in the fraction containing myofibroblasts [CD45(-) PDGFR-α(+) cells] (Figure 2C). These confirmed that the sorting was performed properly. Then, when the expression level of GPR176 in each fraction was measured, it was shown that GPR176 was hardly expressed in the leukocytes (Hem), but strongly expressed in the cell fractions containing the myofibroblasts (Myo) (Figure 2D) . However, in recent years, it has been reported that the expression level of a surface marker PDGFRα for all fibroblasts is decreased along with the differentiation into myofibroblasts (Kanisicak, O. et al. Nat. Commun. 7, 1-14 (2016)), and thus the PDGFRα-positive cells could be a part of the myofibroblast population. Therefore, the reexamination was performed by harvesting the myofibroblast fractions by negative sorting of the CD45(-), CD31(-) as used in the harvest of the fibroblast fraction containing myofibroblasts (Kanisicak, O. et al., Nat. Commun. 7, 1-14 (2016)) (Figure 2E). Cells were isolated by enzyme treatment, and cultured overnight, followed by the removal of the non-adherent cells. Then, the CD45(-) CD31(-) cells (fibroblasts containing myofibroblasts: Myo) and the CD45(+) cells (leukocytes: Hem) were each fractionated using MACS, and the RNAs were extracted from each of the cells. When the expression of Cd68, Acta2 and Colla1 using real-time RT-PCR method was examined similarly to the above experiment, it was shown that each cell fraction was properly harvested (Figure 2F) . Then, when the expression level of GPR176 in each fraction was measured, it was also confirmed that GPR176 was highly expressed on myofibroblasts [CD45(-) CD31(-) cells] (Figure 2G). Overall, Figure 2 shows that GPR176 is highly expressed on myofibroblasts.

### Example 4

### Comparison between GPR176 and α-SMA

The mouse heart sections were used to perform simultaneously the detection of the GPR176 mRNA by in situ hybridization, and the immuno-staining with an antibody against a marker molecule for myofibroblasts α-SMA (Thermo Fisher scientific).

Figures 3A to 3C are fluorescence micrographs showing the examination results. The scale bar is 50 µm. Figure 3A is a micrograph showing the result of the GPR176 mRNA detected, Figure 3B is a micrograph showing the result of the detected α-SMA, and Figure 3C is a micrograph obtained by merging Figures 3A and 3B. In Figures 3A to 3C, the white arrows indicate the position where α-SMA was detected, the black arrows indicate the position where the GPR176 mRNA was detected, and "bv" represents the blood vessel.

As a result, the signals of the GPR176 mRNA were observed in the α-SMA-positive myofibroblasts. It is known that α-SMA is a marker molecule for myofibroblasts, and is also highly expressed in vascular smooth muscle cells. In fact, as shown in Figures 3A to 3C, when observed in a field containing both myofibroblasts and vascular smooth muscle cells, it was shown that the vascular smooth muscle cells arranged in a ring around the blood vessel express α-SMA extremely strongly and that the signal intensity was stronger than that in the myofibroblasts.

Interestingly, the signal of GPR176 mRNA was hardly observed in the α-SMA positive vascular smooth muscle cells. Therefore, it was found that, unlike α-SMA, GPR176 was not expressed on the vascular smooth muscle cells, but was specifically expressed only on myofibroblasts. This indicates that GPR176 is a true myofibroblast-specific marker molecule superior to α-SMA as a marker molecule for myofibroblasts.

### Example 5

### Effect of GPR176 deficiency on collagen accumulation after the myocardial infarction

GPR176 knockout mice were prepared and the effect of GPR176 deficiency on collagen accumulation after the myocardial infarction was investigated. The GPR176 knockout mice showed no significant phenotypic changes under normal conditions as compared with the wild-type control mice. In addition, the GPR176 knockout mice showed no difference in the cardiac functions under normal conditions as compared with the wild-type control mice.

Subsequently, the GPR176 knockout mice (n = 6) and the wild-type control mice (n = 6) were subjected to the same myocardial infarction treatment as in Example 2, and the hearts were removed 28 days after the myocardial infarction treatment. The degree of cardiac fibrosis in each group was estimated by the picrosirius red staining for collagen.

Figure 4A is photographs showing the results of the picrosirius red staining of the heart tissue sections from the wild-type mice (WT) and the GPR176 knockout mice (GPR176 KO) . Figure 4B is a graph of the results of Figure 4A as estimated in numerical form. As a result, it was shown that the GPR176 knockout mice had significantly reduced fibrosis after the myocardial infarction treatment as compared with the control mice.

This result indicates that GPR176 is a protein that promotes fibrosis, and that a neutralizing antibody or an inhibitor against GPR176 would be a new anti-fibrotic agent.

### Example 6

### Effect of GPR176 deficiency on cardiac functions after the myocardial infarction

Fibrosis reduces the function of heart. For example, it has been reported that the cardiac function of the periostin-KO mice after the myocardial infarction is improved since the fibrosis is suppressed (Oka, T. et al. Circ. Res. 101, 313-321 (2007)). In the light of the fact that the fibrosis was suppressed after the infarction in the GPR176-KO mice, we investigated whether or not the suppression of fibrosis caused an improvement in the cardiac function in the GPR176-KO mice. The cardiac functions in the hearts of the wild-type (WT) mice and the GPR176-KO mice 28 days after the infarction treatment were morphologically measured by echocardiography. As a result, the significant improvements in the systolic left ventricular diameter (LVIDs), the left ventricular Ejection Fraction (EF), and the left ventricular diameter shortening rate (FS) were observed in the GPR176-KO mice as compared with the WT mice (Figure 5A). In addition, when the heart weight (HW/BW) and the lungs weight (LW/BW) after the infarction treatment were measured in the light of these results, the increase in the weights after the infarction treatment was significantly reduced in the GPR176-KO mice as compared with the WT mice (Figure 5B). The above results show that GPR176 is a molecule that promotes the fibrosis after the myocardial infarction and that worsens the cardiac function.

### Example 7

### Suppression of expression induction of fibrosis-related factors after the myocardial infarction due to GPR176 deficiency

In the light of the fact that GPR176 promoted the fibrosis in the in vitro experiments, we prepared the GPR176-KO mice to investigate whether or not GPR176 was responsible for fibrosis on individual level. First, the expression levels of the fibrosis-related factors in the hearts of the wild-type (WT: control) mice and the GPR176-KO mice 7 days after the infarction treatment at the time when the expression level of GPR176 peaked, were measured. The measurements were performed by real-time RT-PCR on the total RNAs extracted from the sham-treated ventricle (Sh) and the infarcted region (In) or the region away from the infarcted region of the infarcted heart (Re). As a result, it was confirmed that the expression levels of the fibrosis-promoting factors CTGF (Ctgf), fibronectin (Fn1) and periostin (Postn) were significantly increased by the infarction treatment in both WT and KO mice (Figure 6A). However, their expression induction was significantly attenuated in the GPR176-KO mice as compared with the WT mice (Figure 6A). As described above, myofibroblasts not only perform fibrosis, but also produce the humoral factors and the like after the myocardial infarction and during the pathological condition of cardiac hypertrophy due to hypertension, thereby affecting the hypertrophy of myocardial cells. Thus, we examined the effect of GPR176 deficiency in myofibroblasts on the expressions of these humoral factors and the hypertrophy of cardiomyocytes after the infarction treatment. Specifically, the expression levels of the marker molecules for cardiac hypertrophy ANP (Nppa) and BNP (Nppb), and the cardiac hypertrophy promoting factor IGF-1 (Igf1) were measured in the heart 7 days after the infarction treatment. As a result, the expression levels were significantly increased by the infarction treatment, and the increased expression was significantly attenuated in the GPR176-KO mice (Figure 6B) . From these results, it was considered that the deficiency of GPR176 suppressed the production of IGF-1 from myofibroblasts, and suppressed the hypertrophy of cardiomyocytes. From the above results, it is believed that GPR176 could promote fibrosis through the induction of expression of the fibrosis-related factors, and promote the cardiac hypertrophy after the myocardial infarction, thereby worsening the pathological conditions.

### Example 8

### In vivo studies

It is believed that the expression of GPR176 on myofibroblasts would also promote fibrosis even in vivo. Thus, we investigated whether or not the expressions of fibrosis-related factors were actually reduced on the myofibroblasts of the GPR176 knockout mice.

Cardiac cells were isolated from the hearts of the two wild-type (WT) mice and two GPR176 knockout mice 3 days after the myocardial infarction by collagenase treatment, and then immediately, the cells were stained with the antibodies (Biolegend) against CD45 which is a marker molecule for leukocytes, and Thy1.2 which is a marker molecule for all fibroblasts. Subsequently, the cell sorter (type "FACSAria", BD Biosciences) was used to sort the cell fraction of the CD45(-)Thy1.2(+) containing the myofibroblasts.

Figures 7A and B are graphs showing the sorting of the cell fractions of the CD45(-)Thy1.2(+) from the wild-type mice and the GPR176 knockout mice.

Subsequently, the expression levels of the fibrosis-related factors in the CD45(-)Thy1.2(+) cell fractions recovered from the wild-type mice and the GPR176 knockout mice were compared. The expression levels were measured by real-time RT-PCR. "Viability Dye" which is a dye for determining dead cells is practically a Fixable viability dye eFluor780, and it was obtained from eBiosciences.

Figure 8 is graphs showing the measurement results of the expression levels of the fibrosis-related factors.

As a result, it was observed that the expression levels of the fibrosis-related factors such as α-SMA (Acta2), Colla1 (Colla1), CTGF (Ctgf), and periostin (Postn) tended to decrease in the GPR176 knockout mice as a whole. Therefore, it was believed that GPR176 expressed by the myofibroblasts could promote the expression of the fibrosis-related factors after the myocardial infarction.

### Example 9

### Myofibroblast-specific GPR176 deficiency suppresses expressions of fibrosis-related factors after myocardial infarction

In Examples 1-9, it was revealed that GPR176 was specifically expressed on myofibroblasts and promoted the fibrosis after the myocardial infarction. To support these results, Postn-cre mice 18 expressing Cre recombinase specifically in myofibroblasts, which were often used in recent years in the field of myofibroblast research in the heart and GPR176 flox/flox mice were crossbred to prepare the Postn-cre;GPR176flox/flox mice in which GPR176 is deficient specifically on the myofibroblasts (Figure 9A). Then, we investigated whether or not fibrosis after the myocardial infarction was also suppressed as well in these mice. The experiments were performed using the Postn+/+;GPR176flox/flox mice as control (Ctrl) mice (hereinafter, Ctrl mice) and the Postn+/cre;GPR176flox/flox mice as GPR176 conditional knockout (cKO) mice (hereinafter, GPR176cKO mice).

First, the expression levels of the fibrosis-related factors in the hearts of the Ctrl mice and the GPR176cKO mice 7 days after the infarction treatment were measured in the same manner as in the examination using the mice systemically deficient with GPR176. The measurements were performed by real-time RT-PCR on the total RNAs extracted from the sham-treated ventricle (Sh) and the infarcted region (In) or the region away from the infarcted region of the infarcted heart (Re). As a result, the expression levels of the fibrosis-promoting factors CTGF (Ctgf), fibronectin (Fn1), and periostin (Postn) were significantly increased by the infarction treatment in both Ctrl and cKO mice (Figure 9B). However, their expression inductions were significantly attenuated in the GPR176cKO mice as compared with the Ctrl mice (Figure 9B). In addition, the expression levels of cardiac hypertrophy markers ANP (Nppa) and BNP (Nppb), and a cardiac hypertrophy promoting factor IGF-1 (Igf1), were also significantly increased by the myocardial infarction treatment in both Ctrl and cKO mice (Figure 9C). However, the induction of IGF1 (Igf1) expression was significantly attenuated in the GPR176cKO mice as compared with the Ctrl mice, and the expressions of ANP (Nppa) and BNP (Nppb) tended to decrease in the cKO mice (Figure 9B). From the above results, it was shown that GPR176 promotes the induction of expression of the fibrosis-related factors on myofibroblasts, and is responsible for fibrosis in hearts.

### Example 10

### Myofibroblast-specific GPR176 deficiency suppresses fibrosis after the myocardial infarction

In the light of the fact that a decrease in the expression levels of the fibrosis-related factors after the myocardial infarction treatment was also observed in the mice in which GPR176 is deficient specifically in the myofibroblasts, we also investigated the effects of the myofibroblast-specific GPR176 deficiency on the survival and the progression of fibrosis after the myocardial infarction treatment. The Ctrl mice and the GPR176cKO mice were treated for myocardial infarction, and the survival rate was examined 28 days after the treatment. As a result, no difference in survival rate between the Ctrl mice and the GPR176cKO mice was observed (Figure 9D).

Next, the picrosirius red staining for dyeing collagen red was performed on the Ctrl mouse hearts and the GPR176cKO mouse hearts 28 days after the infarction treatment, to evaluate the degrees of fibrotic region after the infarction treatment (Figure 9E). Figure 9F shows the actual quantification of the volume of the collagen accumulated (CVF; Collagen Volume Fraction). CVS was determined by counting the collagen deposit areas. This result demonstrated that the volume of the collagen accumulated in the GPR176cKO mice was significantly reduced as compared with that in the Ctrl mice. Overall, Figure 9 shows that the myofibroblast-specific GPR176 deficiency reduces fibrosis after the myocardial infarction.

From the above results, it was shown that the function of GPR176 to promote the expression of the fibrosis-related factors and to exacerbate fibrosis is likely to be mediated by the myofibroblasts.

### Example 11

### Myofibroblast-specific GPR176 deficiency suppresses fibrosis after the myocardial infarction

In the light of the fact that fibrosis after the myocardial infarction treatment was suppressed in the GPR176cKO mice, we investigated whether or not the improvement in cardiac function was observed in association with the suppression of fibrosis in the GPR176cKO mice.

Specifically, the cardiac functions in the heart of the Ctrl mice and the GPR176cKO mice 28 days after the myocardial infarction were morphologically measured by echocardiography. As a result, the left ventricular Ejection Fraction (EF) and the left ventricular diameter shortening rate (FS) were significantly improved in the cKO mice in the same manner as in the mice systemically deficient with GPR176 (Figure 10A). In addition, the heart weight (HW/BW) and the lungs weight (LW/BW) after the infarction treatment were measured, but no significant differences were observed in the weight gains after the infarction treatment between the Ctrl mice and the cKO mice (Figure 10B). Overall, Figure 10 shows that the myofibroblast-specific GPR176 deficiency improves the cardiac functions after the infarction treatment.

The above results demonstrated that GPR176 expressed in myofibroblasts is responsible for the promotion of fibrosis in vivo.

### Example 12

### Examination in the liver

It was examined whether or not myofibroblasts prominently express GPR176 even during the liver pathology. In the liver, myofibroblasts are called Hepatic Stellate Cells (HSC). Hepatic stellate cells overproduce extracellular matrix proteins such as collagen to perform the fibrosis. Thus, the expression levels of GPR176 in hepatic stellate cells (HSC), hepatocytes constituting the liver (HC) and kupffer cells (KC), which are the resident macrophages of the liver, were compared.

Specifically, the livers of the mice administered with carbon tetrachloride were treated with collagenase to disperse the cells, and then each cell fraction of HSC, HC, and KCs was harvested by centrifugation and MACS magnetic cell separation.

Subsequently, the expression levels of the marker molecules for myofibroblast, the marker molecule for hepatocyte, and the marker molecule for macrophage in each cell fraction were measured. The expression level was measured by real-time RT-PCR. The results are shown in Figure 11A.

Figure 11A shows that the marker molecules for myofibroblast α-SMA (Acta2) and Col1a1 (Col1a1) were significantly expressed only in the hepatic stellate cell (HSC) fraction. It was also confirmed that the marker molecule for hepatocyte, Cyp7a1 (Cyp7a1) was significantly expressed only in the hepatocyte (HC) fraction. It was also confirmed that the marker for macrophage, CD68 (Cd68) was significantly expressed only in the Kupffer Cell (KC) fraction. These results indicated that each cell was properly sorted.

Thus, the same cell samples were used to measure the expression level of GPR176. Figure 11B is a graph showing the measurement results of the expression level of GPR176. The gene expression level is shown as a relative value to the GAPDH gene expression level.

As a result, it was shown that GPR176 was significantly expressed in the hepatic stellate cell (HSC) fraction. Therefore, this result shows that GPR176 is highly expressed in hepatic stellate cells (myofibroblasts) which perform fibrosis even during liver fibrosis, and it is confirmed that GPR176 is closely related to the pathological condition of liver fibrosis.

### Example 13

### Expression of GPR176 in liver fibrosis model mice

We investigated whether or not the increased expression of GPR176 was observed in the liver fibrosis. It is known that the administration of carbon tetrachloride to mice for 4 weeks induces the liver fibrosis, and, with stopping the administration of carbon tetrachloride, the continued raising for 4 weeks eliminates the liver fibrosis. Thus, this liver fibrosis model was used to examine the expression levels of α-SMA and GPR176.

Figure 12A is a graph showing the result of the quantified expression level of α-SMA(Acta2) by real-time RT-PCR in the livers of the mice of the control group not receiving carbon tetrachloride (n = 4-6), the group receiving carbon tetrachloride for 4 weeks (n = 6), and the group receiving carbon tetrachloride for 4 weeks, and then, with stopping the administration of carbon tetrachloride, continuing raising for 4 weeks (n = 5).

In addition, Figure 12B is a graph showing the result of the quantified expression level of GPR176 by real-time RT-PCR in the livers of the mice of the control group not receiving carbon tetrachloride (n = 4-6), the group receiving carbon tetrachloride for 4 weeks (n = 6), and the group receiving carbon tetrachloride for 4 weeks, and then, with stopping the administration of carbon tetrachloride, continuing raising for 4 weeks (n = 5).

As a result, it was shown that the expression levels of α-SMA and GPR176 were significantly increased by the administration of carbon tetrachloride. In addition, it was shown that the expression levels of α-SMA and GPR176 were decreased when carbon tetrachloride was administered for 4 weeks, the administration was stopped, and then the animals were raised for 4 weeks. This result indicates that GPR176 is also responsible for the pathological condition of fibrosis in the liver.

### Example 14

### Expression of GPR176 in non-alcoholic steatohepatitis model mice

The NASH model mice have been known, which develop a NASH-like pathology when C57BL/6J mice is fed with "A06071302 (60 Kcal% fat)" (Ultra High Fat Choline Deficient Methionine Weight Loss Feed, Research Diets), a diet for generating non-alcoholic steatohepatitis (NASH), for 5 weeks.

Thus, this model mouse was used to examine the expression level of GPR176 in the NASH-like pathology. In addition, the expression level of COl1a1 that is a marker for NASH, was examined for comparison. The expression level was measured by real-time RT-PCR.

Figure 13A is a graph showing the results of the expression level of COl1a1 quantified by real-time RT-PCR in the livers of the control mice (n = 5) receiving a normal diet and the NASH model mice (n = 5) receiving the diet for generating NASH. In addition, Figure 13B is a graph showing the results of the expression level of GPR176 quantified by real-time RT-PCR in the livers of the control mice (n = 5) receiving a normal diet and the NASH model mice (n = 5) receiving the diet for generating

### NASH.

As a result, it was shown that the expression levels of COl1a1 and GPR176 were significantly increased in the NASH model mice. This result indicates that GPR176 is responsible for the liver fibrosis in a NASH-like pathology.

### Example 15

### GPR176 expression is also increased during lung and renal fibrotic conditions

Fibrosis of organs is observed not only in the heart but also in various organs such as the kidneys, the liver and the lungs. Thus, we investigated whether or not the expression level of GPR176 increases even during fibrotic pathology of organs other than the heart.

Idiopathic pulmonary fibrosis, a fibrotic disease of the lung, is said to cause death in about half of patients within 2 to 5 years after the diagnosis (Carrington, R. Jordan, S. Pitchford, S.C. & Page, C.P., Pulm. Pharmacol. Therauptics 51, 73-78 (2018)), and the development of new therapies therefor is urgent. Bleomycin (BLM) is a chemotherapeutic agent used to treat several neoplastic diseases such as lymphoma, head and neck squamous cell carcinoma, testicular cancer, and ovarian cancer. BLM produces reactive oxygen species (ROS) and reactive nitrogen species (RNS) in the presence of iron and oxygen. These result in the DNA strand breaks, and induce the acute interstitial and alveolar inflammation, thereby inducing the fibrosis. The BLM-administered pulmonary fibrosis model is the most widely used method for studying the pulmonary fibrosis in animal models (Bleomycin in the setting of lung fibrosis induction: From biological mechanisms to counteractions. PMID: 25959210 https://www.ncbi.nlm.nih.gov/pubmed/25959210).

In order to investigate the expression of GPR176 during the pulmonary fibrosis, we attempted to create a pulmonary fibrosis model by administering a single dose of BLM through the trachea (Figure 14A). When the expression levels of the fibrosis-related factors in the lungs 14 days after the BLM administration were measured by real-time RT-PCR, the bleomycin-administered group showed significant increase in the expression levels of Acta2, Col1a1, and Ctgf, as compared with the saline (Saline)-administered group as a control, confirming that the pulmonary fibrosis was properly induced by the BLM administration (Figure 14B). When the same sample was used to measure the expression level of GPR176, the significant increase in the expression of GPR176 was observed in the BLM-administered group (Figure 14C). Therefore, it was suggested that GPR176 could be responsible for fibrosis not only in the heart but also in the lungs.

On the other hand, chronic renal failure is found in about half of adults aged 70 years or older, and generates fibrosis at the time of its pathology, which contributes to renal dysfunction (Humphreys, B.D. Mechanisms of Renal Fibrosis. (2018). https://www.ncbi.nlm.nih.gov/pubmed/29068765). Thus, fibrosis in kidney is also actively studied like in the lungs. Unilateral Ureteral Obstruction (UUO) induces fibrosis in the renal stroma by ligating the ureter on one side to inhibit urine excretion, which causes changes in hemodynamics due to mechanical stretching such as tubular dilation and interstitial dilation, and inflammation due to the apoptosis of renal tubules epithelial cells and oxidative stress (Martinez-Klimova, E., Aparicio-Trejo, O. E., Tapia, E. & Pedraza-Chaverri, J., Biomolecules 9, (2019)). In order to investigate the expression of GPR176 during the renal fibrosis pathology, we attempted to prepare a renal fibrosis model using UUO (Figure 14D). Renal fibrosis was induced by ligating the right ureter. Sham-treatment was used as a control. When the expression levels of the fibrosis-related factors in the kidney 7 days after the UUO treatment were measured by real-time RT-PCR, it was shown that the expression levels of a marker molecule for myofibroblasts α-SMA (Acta2), and the marker molecules for fibrosis Col1a1 (Col1a1) and CTGF (Ctgf) were significantly increased in the UUO group as compared with those in the sham-treatment group, confirming that fibrosis was induced therein (Figure 14E). When the same sample was used to measure the expression level of GPR176, it was revealed that the expression level was significantly increased in the kidneys of the UUO group as compared with that in the sham-treatment group (Figure 14F). Overall, Figure 14 shows that the expression level of GPR176 is also increased during the lung and renal fibrotic conditions.

These results suggest that GPR176 could be responsible for fibrosis not only in the heart but also in other organs such as the lungs and kidneys.

### Example 16

### GPR176 is expressed on myofibroblasts even during fibrotic pathology in lungs

We investigated whether or not GPR176 is expressed on myofibroblasts in the lung as well as in the heart. First, in situ hybridization method on the normal lungs was performed to detect the GPR176 mRNA, and no expression of GPR176 was observed in the normal tissue in the lungs as well as in the heart (Figure 15A). Next, against the lung sections of the mice in which fibrosis was induced by administrating BLM, the detection of GPR176 mRNA by in situ hybridization and the immunostaining using antibodies against the various cell markers were performed at the same time. The GPR176 signal was rarely observed in the CD31-positive endothelial cells and the CD45-positive leukocytes, but observed only in the αSMA-positive myofibroblasts.

### Example 17

### GPR176 promotes the expression of fibrosis-related factors on myofibroblasts

In the light of the fact that GPR176 is strongly expressed on myofibroblasts, we investigated whether or not GPR176 regulates the expression of the fibrosis-related factors on myofibroblasts.

First, the siRNA against GPR176 (siGPR176) was introduced into the myofibroblasts isolated from the mouse heart treated by the myocardial infarction treatment, and when GPR176 was thereby knocked down, real-time RT-PCR was performed to investigate whether or not the expression levels of the fibrosis-related factors were changed. Specifically, the cardiac myofibroblasts were isolated from the infarcted heart of the WT mice 3 days after the infarction treatment, and then the siRNA against GPR176 was transfected into the isolated cardiac myofibroblasts. 96 hours after the transfection, the total RNAs were extracted from these cells, and were subjected to real-time RT-PCR. When the expression level of GPR176 was measured, it was shown that the expression of GPR176 mRNA in the cardiac myofibroblasts treated with the siRNA against GPR176 was decreased. It was confirmed that the GPR176 expression was suppressed to about 30% by the introduction of the siGPR176 (Figure 16A). Under these conditions, the expression levels of the fibrosis-promoting factors CTGF (Ctgf), fibronectin (Fn1), periostin (Postn), and TGF-β2 (Tgfb2) were significantly decreased in the siGPR176-introduction group (Figure 16A).

As a control siRNA, Silencer^{™} Select Negative Control No. 1 siRNA #4390843 sequence was used (ThermoFischer). The siRNA against GPR176 was Silencer^{™} Select siRNA #117673, wherein these sequences were as follows: Sense: GAUAUUUCCUGAUAAGUAUtt (SEQ ID NO: 9 of the Sequence Listing), and Antisense: AUACUUAUCAGGAAAUAUCcc (SEQ ID NO: 10 of the Sequence Listing) (Thermo Fischer).

Myofibroblasts not only perform fibrosis, but also produce the humoral factors and the like after the myocardial infarction and during the pathological condition of cardiac hypertrophy due to hypertension, thereby affecting the hypertrophy of myocardial cells (Takeda, N. & Manabe, I., Int. J. Inflam. 2011, 1-13 (2011)). Thus, we also investigated the effect of knockdown of GPR176 on the expression of these humoral factors on myofibroblasts. Specifically, the expression level of IGF-1 (Igf1), which acts on myocardial cells and causes cardiac hypertrophy, was examined, and it was found that the expression level was significantly decreased in the siGPR176-introduced group (Figure 16A).

Next, contrary to the knockdown, GPR176 was overexpressed on myofibroblasts isolated from the mouse heart after the myocardial infarction treatment using retroviruses, and it was evaluated by real-time RT-PCR whether or not the expression level of the fibrosis-related factors was increased. Specifically, the cardiac myofibroblasts were isolated from the infarcted heart of the WT mice on the third day after the infarction treatment, and then the isolated cardiac myofibroblasts were infected with the retrovirus of GPR176. 48 hours after the infection, the total RNAs were extracted from these cells, and were subjected to real-time RT-PCR. The result showed that the expression levels of the fibrosis-promoting factors CTGF (Ctgf), periostin (Postn), and TGF-β2 (Tgfb2) were significantly increased by the GPR176 overexpression (Figure 16B). In addition, the expression level of fibronectin (Fn1) tended to increase due to the GPR176 overexpression (Figure 16B). Further, the expression level of a cardiac hypertrophy promoting factor IGF-1 (Igf1) was also significantly increased by the GPR176 overexpression (Figure 16B). Overall, Figure 16 shows that GPR176 promotes the expression of the fibrosis-related factors on myofibroblasts.

From the results of the above GPR176 knockdown and overexpression experiments in myofibroblasts, it was shown that GPR176 expressed on myofibroblasts is a molecule that promotes fibrosis.

### Example 18

We investigated in detail whether or not the cells expressing GPR176 express α-SMA.

In situ hybridization method was used to detect the GPR176 mRNA on the mouse heart sections. In situ hybridization method enables to detect the expressed mRNA of the target molecule in dots by using a probe specific for that molecule. Co-detection of the GPR176 mRNA and the α-SMA protein was performed on heart sections of the mouse 7 days after the myocardial infarction treatment. As a result, it was shown that almost all of the GPR176-positive cells express α-SMA in the border of the infarcted region (Border) and the infarcted region (Infarct) of the mouse heart. The results are shown in Figure 17. The ratios of the α-SMA expression in the GPR176-positive cells and the GPR176 expression in the α-SMA-positive cells were estimated in 12 visual fields on one side (24 visual fields in total) of the border zone and 11 visual fields of the infarct region per section. The data of three independent sections were averaged and expressed as mean ± standard error. The scale bar is 20 µm.

### Example 19

### Relationship between cells expressing GPR176 in the heart and cells expressing conventional myofibroblast markers

To examine the validity of GPR176 as a marker for myofibroblasts, the expression of GPR176 was compared with the expression of the conventional markers for myofibroblasts.

αSMA has been often used as a marker for myofibroblasts in the past, but, in recent years, a molecule periostin (Postn) has been reported as a more specific marker for the heart (Kanisicak, O. et al. Nat. Commun. 7, 1-14 (2016)). Thus, the myofibroblasts [CD45(-), CD31(-) cells] isolated from the heart of the infarcted mice were used simultaneously to detect the GPR176 mRNA and the Postn mRNA by in situ hybridization, and to perform the immunostaining using the αSMA-antibody, and their expressions were examined (Figure 18A). Specifically, the number of the GPR176 mRNA-positive cells in the αSMA-positive cells and the Postn-positive cells was estimated, and the number of the αSMA-positive cells or the Postn-positive cells in the GPR176 mRNA-positive cells was estimated. The number of the GPR176 mRNA-positive cells in the αSMA-positive cells and the Postn-positive cells was also estimated in the same manner. As a result, about 99% of each marker-positive cells were the GPR176 mRNA-positive cells (Figure 18C). Therefore, it was revealed that most of the cells expressing the conventional myofibroblast markers express the GPR176 mRNA.

On the other hand, the marker-positive cells for all markers in the GPR176 mRNA-positive cells were at about 98% (Figure 18D, E). Therefore, it was revealed that most of the GPR176 mRNA-positive cells were myofibroblast marker-positive cells. Similar studies were also performed on leukocytes [CD45-positive cells] (Figure 18B), but few GPR176 mRNA-positive cells were observed (Figure 18C). Overall, Figure 18 shows that the GPR176 expression is highly correlated with the expression of the conventional cardiac myofibroblast markers (a-SMA and periostin).

Therefore, the GPR176-expressing cells were almost the same as the conventional myofibroblast marker-expressing cells, and it was believed that GPR176 could be a true myofibroblast marker.

### Example 20

### GPR176-expressing cells closely match the conventional myofibroblast marker-expressing cells in the lung as well

Next, in order to investigate whether or not GPR176 was appropriate as a marker for myofibroblasts not only in the heart but also in the lungs, the myofibroblasts [CD45(-), CD31(-), CD326(-) cells] were isolated from the lungs of the BLM-administered mice (Figure 19A), and these cells were used simultaneously to detect the GPR176 mRNA and the Postn mRNA by in situ hybridization, and to perform the immunostaining using the αSMA-antibody as the same manner in the examination in the heart, thereby examining their co-localization (Figure 19B). First, the number of the GPR176 mRNA-positive cells in the αSMA-positive cells was estimated. As a result, it was revealed that about 97% of the αSMA-positive cells were the GPR176-positive cells (Figure 19D). Conversely, the number of the αSMA-positive cells in the GPR176-positive cells was also estimated in the same manner, and it was revealed that 98% of the GPR176-positive cells were the αSMA-positive cells (Figure 19E). These results showed that GPR176 could be a marker receptor for myofibroblasts not only in the heart but also in the lungs. A similar study was also performed on the leukocytes [CD45-positive cells], but few GPR176 mRNA-positive cells were observed (Figure 19B, D). Overall, Figure 19 shows that the GPR176-expressing cells closely match the cells expressing the conventional myofibroblast marker α-SMA in the lung as well.

Therefore, GPR176 is expressed in almost all myofibroblasts in the lung, and it was believed that GPR176 could be a marker for true myofibroblasts in the lung as well.

### Example 21

### GPR176 is also specifically expressed on myofibroblasts in the human heart

In order to investigate whether or not GPR176 was expressed in the myofibroblasts not only in mice but also in humans, the detection of GPR176 mRNA by in situ hybridization and the immunostaining with an antibody against αSMA were simultaneously performed on the cardiac sections of the patients suffered from myocardial infarction and the patients not suffered from myocardial infarction. As a result, almost no signal of GPR176 was observed in the cardiac section of the patients not suffered from myocardial infarction (Figure 20A). On the other hand, the signal of GPR176 in the heart section of the patients suffered from myocardial infarction was observed in the αSMA-positive myofibroblasts (Figure 20B). Therefore, it was shown that GPR176 was not expressed in the heart where fibrosis has not developed, but was expressed in the myofibroblasts appearing during fibrosis not only in the mouse heart but also the human heart.

### Example 22

### Most of the labeled cells in the heart of GPR176 reporter mice after infarction treatment are myofibroblasts.

In the light of the fact that GPR176 could be a novel marker for myofibroblasts not only in the heart but also in various organs, a genetically modified mouse GPR176-cre mouse in which Cre was knocked in the downstream of the GPR176 promoter was prepared, and this mouse was crossbred with a Rosa26-tdTomato mouse (Swonger, J.M., Liu, J.S., Ivey, M.J. & Tallquist, M. D., Differentiation 92, 66-83 (2016)), to prepare GPR176 reporter mice in which the GPR176-expressing cells were labeled with tdTomato (Figure 21A). Infarct treatment was performed on these mice, and the tdTomato-labeled cells in the heart sections of mice 3 days after the infarction were examined by immunohistochemical staining. As a result, the tdTomato-labeled cells were found in the interstitium. Further, it was confirmed that most of them were αSMA-positive, while they were negative for CD68, a marker for monocytes and macrophages (Figure 21B).

### INDUSTRIAL APPLICABILITY

According to the present invention, it is possible to identify a marker protein for myofibroblasts, and provide a modality for preventing or treating fibrotic diseases.

## Claims

1. A pharmaceutical composition for preventing or treating fibrotic diseases, which comprises an inhibitor of G protein-coupled receptor 176 (GPR176) as an active ingredient.

2. The pharmaceutical composition according to claim 1, wherein the inhibitor of GPR176 is an inhibitor of the GPR176 expression or an inhibitor of the GPR176 function.

3. The pharmaceutical composition according to claim 2, wherein the inhibitor of GPR176 is an inhibitor of the GPR176 expression.

4. The pharmaceutical composition according to claim 2, wherein the inhibitor of the GPR176 expression is a substance that inhibits the expression of the gene or nucleic acid represented by any one of (a) to (c):
(a) the GPR176 gene set forth in any one of SEQ ID NOs: 2, 4 or 6 of the Sequence Listing,
(b) a nucleic acid that comprises a base sequence containing a deletion, a substitution or an addition of one or several bases in the GPR176 gene set forth in any one of SEQ ID NOs: 2, 4 or 6 of the Sequence Listing, and
(c) a nucleic acid that hybridizes under stringent conditions with a base sequence complementary to the GPR176 gene set forth in any one of SEQ ID NOs: 2, 4 or 6 of the Sequence Listing.

5. The pharmaceutical composition according to claim 4, wherein the substance that inhibits the expression of the gene or nucleic acid is selected from the group consisting of an siRNA, an antisense and a ribozyme.

6. The pharmaceutical composition according to claim 2, wherein the inhibitor of GPR176 is an inhibitor of the GPR176 function.

7. The pharmaceutical composition according to claim 6, wherein the inhibitor of the GPR176 function is a substance specifically binding to the GPR176 protein.

8. The pharmaceutical composition according to claim 7, wherein the substance specifically binding to the GPR176 protein is selected from the group consisting of an antibody, an antibody fragment and an aptamer.

9. The pharmaceutical composition according to claim 8, wherein the antibody fragment is selected from the group consisting of Fv, Fab and scFv.

10. A nucleic acid selected from the group consisting of an siRNA, an antisense and a ribozyme, which inhibits the expression of:
(a) the GPR176 gene set forth in any one of SEQ ID NOs: 2, 4 or 6 of the Sequence Listing,
(b) a nucleic acid that comprises a base sequence containing a deletion, a substitution or an addition of one or several bases in the GPR176 gene set forth in any one of SEQ ID NOs: 2, 4 or 6 of the Sequence Listing, or
(c) a nucleic acid that hybridizes under stringent conditions with a base sequence complementary to the GPR176 gene set forth in any one of SEQ ID NOs: 2, 4 or 6 of the Sequence Listing.

11. A vector comprising the gene or nucleic acid according to claim 10.

12. A cell comprising the vector according to claim 11.

13. A method of screening for an inhibitor of GPR176, which comprises confirming that the expression of a gene or a nucleic acid is inhibited, which is **characterized by** the use of said gene or said nucleic acid represented by any one of (a) to (c):
(a) the GPR176 gene set forth in any one of SEQ ID NOs: 2, 4, 6 or 8 of the Sequence Listing,
(b) a nucleic acid that comprises a base sequence containing a deletion, a substitution or an addition of one or several bases in the GPR176 gene set forth in any one of SEQ ID NOs: 2, 4, 6 or 8 of the Sequence Listing, or
(c) a nucleic acid that hybridizes under stringent conditions with a base sequence complementary to the GPR176 gene set forth in any one of SEQ ID NOs: 2, 4, 6 or 8 of the Sequence Listing, or
the use of a cell into which the gene or nucleic acid has been introduced.

14. The method of screening for an inhibitor of GPR176 according to claim 13, which comprises;
(1) preparing a candidate compound,
(2) contacting said candidate compound to a cell comprising the gene or nucleic acid represented by any one of: ,
(a) the GPR176 gene set forth in any one of SEQ ID NOs: 2, 4, 6 or 8 of the Sequence Listing,
(b) a nucleic acid that comprises a base sequence containing a deletion, a substitution or an addition of one or several bases in the GPR176 gene set forth in any one of SEQ ID NOs: 2, 4, 6 or 8 of the Sequence Listing, or
(c) a nucleic acid that hybridizes under stringent conditions with a base sequence complementary to the GPR176 gene set forth in any one of SEQ ID NOs: 2, 4, 6 or 8 of the Sequence Listing, and
(3) determining whether or not said candidate compound suppresses the expression of said gene or nucleic acid.

15. The method according to claim 13 or 14, which comprises using the GPR176 gene set forth in any one of SEQ ID NOs: 2, 4 or 6 of the Sequence Listing.

16. A method of screening for a substance that inhibits the function of a protein, **characterized by** the use of said protein, which is the GPR176 protein having the amino acid sequence set forth in any one of SEQ ID NOs: 1, 3, 5 or 7 of the Sequence Listing, or a variant GPR176 protein which contains a substitution, a deletion or an addition of one or several amino acid residues in the GPR176 protein, and which has the same activity as said GPR176 protein.

17. A method of screening for a substance that inhibits the function of the GPR176 protein, which comprises;
(1) preparing a candidate compound,
(2) contacting said candidate compound to the GPR176 protein having the amino acid sequence set forth in any one of SEQ ID NOs: 1, 3, 5 or 7 of the Sequence Listing, or a variant GPR176 protein which contains a substitution, a deletion or an addition of one or several amino acid residues in the GPR176 protein, and which has the same activity as said GPR176 protein, or to a cell expressing said GPR176 protein or said variant GPR176 protein, and
(3) determining whether or not said candidate compound inhibits the function of said GPR176 protein or said variant GPR176 protein.

18. The method according to claim 16 or 17, which comprises using the GPR176 protein having the amino acid sequence set forth in any one of SEQ ID NOs: 1, 3 or 5 of the Sequence Listing, or a variant GPR176 protein which contains a substitution, a deletion or an addition of one or several amino acid residues in the GPR176 protein, and which has the same activity as said GPR176 protein.

19. A method of screening for a prophylactic or therapeutic agent for fibrotic diseases, which comprises a step of culturing myofibroblasts in the presence of a candidate compound, a step of quantifying the expression level of the mRNA of the GPR176 gene or the GPR176 protein in the cultured myofibroblasts, and a step of judging the candidate compound to be a prophylactic or therapeutic agent for fibrotic diseases when the quantified expression level of the mRNA of said GPR176 gene or said GPR176 protein is decreased as compared with that of the control.

20. A method of determining the severity in fibrotic diseases, which comprises;
(a10) a step of measuring the amount of GPR176 on myofibroblasts of a subject (test biomarker amount),
(b10) a step of comparing the amount of the test biomarker with the amount of GPR176 on myofibroblasts of a healthy subject (control biomarker amount), and
(c10) a step of determining that fibrotic diseases become serious when the test biomarker amount is larger than the control biomarker amount.

21. A biomarker that is GPR176 of myofibroblasts which enables to determine the severity in fibrotic diseases.

22. A kit for detecting myofibroblasts, which comprises a primer set for amplifying the cDNA of GPR176, a probe that specifically hybridizes with the mRNA of GPR176, or a substance specifically binding to the GPR176 protein.


SEQUENCE LISTING

<110>  KYUSHU UNIVERSITY, NATIONAL UNIVERSITY CORPORATION

<120>  Prevention or treatment of fibrotic diseases

<130>  675453

<150>  JP 2019-030791
<151>  2019-02-22

<160>  10

<170>  PatentIn version 3.5

<210>  1
<211>  515
<212>  PRT
<213>  human

<400>  1

Met Gly His Asn Gly Ser Trp Ile Ser Pro Asn Ala Ser Glu Pro His
1               5                   10                  15

Asn Ala Ser Gly Ala Glu Ala Ala Gly Val Asn Arg Ser Ala Leu Gly
            20                  25                  30

Glu Phe Gly Glu Ala Gln Leu Tyr Arg Gln Phe Thr Thr Thr Val Gln
        35                  40                  45

Val Val Ile Phe Ile Gly Ser Leu Leu Gly Asn Phe Met Val Leu Trp
    50                  55                  60

Ser Thr Cys Arg Thr Thr Val Phe Lys Ser Val Thr Asn Arg Phe Ile
65                  70                  75                  80

Lys Asn Leu Ala Cys Ser Gly Ile Cys Ala Ser Leu Val Cys Val Pro
                85                  90                  95

Phe Asp Ile Ile Leu Ser Thr Ser Pro His Cys Cys Trp Trp Ile Tyr
            100                 105                 110

Thr Met Leu Phe Cys Lys Val Val Lys Phe Leu His Lys Val Phe Cys
        115                 120                 125

Ser Val Thr Ile Leu Ser Phe Pro Ala Ile Ala Leu Asp Arg Tyr Tyr
    130                 135                 140

Ser Val Leu Tyr Pro Leu Glu Arg Lys Ile Ser Asp Ala Lys Ser Arg
145                 150                 155                 160

Glu Leu Val Met Tyr Ile Trp Ala His Ala Val Val Ala Ser Val Pro

                165                 170                 175

Val Phe Ala Val Thr Asn Val Ala Asp Ile Tyr Ala Thr Ser Thr Cys
            180                 185                 190

Thr Glu Val Trp Ser Asn Ser Leu Gly His Leu Val Tyr Val Leu Val
        195                 200                 205

Tyr Asn Ile Thr Thr Val Ile Val Pro Val Val Val Val Phe Leu Phe
    210                 215                 220

Leu Ile Leu Ile Arg Arg Ala Leu Ser Ala Ser Gln Lys Lys Lys Val
225                 230                 235                 240

Ile Ile Ala Ala Leu Arg Thr Pro Gln Asn Thr Ile Ser Ile Pro Tyr
                245                 250                 255

Ala Ser Gln Arg Glu Ala Glu Leu His Ala Thr Leu Leu Ser Met Val
            260                 265                 270

Met Val Phe Ile Leu Cys Ser Val Pro Tyr Ala Thr Leu Val Val Tyr
        275                 280                 285

Gln Thr Val Leu Asn Val Pro Asp Thr Ser Val Phe Leu Leu Leu Thr
    290                 295                 300

Ala Val Trp Leu Pro Lys Val Ser Leu Leu Ala Asn Pro Val Leu Phe
305                 310                 315                 320

Leu Thr Val Asn Lys Ser Val Arg Lys Cys Leu Ile Gly Thr Leu Val
                325                 330                 335

Gln Leu His His Arg Tyr Ser Arg Arg Asn Val Val Ser Thr Gly Ser
            340                 345                 350

Gly Met Ala Glu Ala Ser Leu Glu Pro Ser Ile Arg Ser Gly Ser Gln
        355                 360                 365

Leu Leu Glu Met Phe His Ile Gly Gln Gln Gln Ile Phe Lys Pro Thr
    370                 375                 380

Glu Asp Glu Glu Glu Ser Glu Ala Lys Tyr Ile Gly Ser Ala Asp Phe
385                 390                 395                 400

Gln Ala Lys Glu Ile Phe Ser Thr Cys Leu Glu Gly Glu Gln Gly Pro
                405                 410                 415

Gln Phe Ala Pro Ser Ala Pro Pro Leu Ser Thr Val Asp Ser Val Ser
            420                 425                 430

Gln Val Ala Pro Ala Ala Pro Val Glu Pro Glu Thr Phe Pro Asp Lys
        435                 440                 445

Tyr Ser Leu Gln Phe Gly Phe Gly Pro Phe Glu Leu Pro Pro Gln Trp
    450                 455                 460

Leu Ser Glu Thr Arg Asn Ser Lys Lys Arg Leu Leu Pro Pro Leu Gly
465                 470                 475                 480

Asn Thr Pro Glu Glu Leu Ile Gln Thr Lys Val Pro Lys Val Gly Arg
                485                 490                 495

Val Glu Arg Lys Met Ser Arg Asn Asn Lys Val Ser Ile Phe Pro Lys
            500                 505                 510

Val Asp Ser
        515

<210>  2
<211>  1548
<212>  DNA
<213>  human

<400>  2
atgggacata acgggagctg gatctctcca aatgccagcg agccgcacaa cgcgtccggc      60
gccgaggctg cgggtgtgaa ccgcagcgcg ctcggggagt tcggcgaggc gcagctgtac     120
cgccagttca ccaccaccgt gcaggtcgtc atcttcatag gctcgctgct cggaaacttc     180
atggtgttat ggtcaacttg ccgcacaacc gtgttcaaat ctgtcaccaa caggttcatt     240
aaaaacctgg cctgctcggg gatttgtgcc agcctggtct gtgtgccctt cgacatcatc     300
ctcagcacca gtcctcactg ttgctggtgg atctacacca tgctcttctg caaggtcgtc     360
aaatttttgc acaaagtatt ctgctctgtg accatcctca gcttccctgc tattgctttg     420
gacaggtact actcagtcct ctatccactg gagaggaaaa tatctgatgc caagtcccgt     480
gaactggtga tgtacatctg ggcccatgca gtggtggcca gtgtccctgt gtttgcagta     540
accaatgtgg ctgacatcta tgccacgtcc acctgcacgg aagtctggag caactccttg     600
ggccacctgg tgtacgttct ggtgtataac atcaccacgg tcattgtgcc tgtggtggtg     660
gtgttcctct tcttgatact gatccgacgg gccctgagtg ccagccagaa gaagaaggtc     720
atcatagcag cgctccggac cccacagaac accatctcta ttccctatgc ctcccagcgg     780
gaggccgagc tgcacgccac cctgctctcc atggtgatgg tcttcatctt gtgtagcgtg     840
ccctatgcca ccctggtcgt ctaccagact gtgctcaatg tccctgacac ttccgtcttc     900
ttgctgctca ctgctgtttg gctgcccaaa gtctccctgc tggcaaaccc tgttctcttt     960
cttactgtga acaaatctgt ccgcaagtgc ttgataggga ccctggtgca actacaccac    1020
cggtacagtc gccgtaatgt ggtcagtaca gggagtggca tggctgaggc cagcctggaa    1080
cccagcatac gctcgggtag ccagctcctg gagatgttcc acattgggca gcagcagatc    1140
tttaagccca cagaggatga ggaagagagt gaggccaagt acattggctc agctgacttc    1200
caggccaagg agatatttag cacctgcctg gagggagagc aggggccaca gtttgcgccc    1260
tctgccccac ccctgagcac agtggactct gtatcccagg tggcaccggc agcccctgtg    1320
gaacctgaaa cattccctga taagtattcc ctgcagtttg gctttgggcc ttttgagttg    1380
cctcctcagt ggctctcaga gacccgaaac agcaagaagc ggctgcttcc ccccttgggc    1440
aacaccccag aagagctgat ccagacaaag gtgcccaagg taggcagggt ggagcggaag    1500
atgagcagaa acaataaagt gagcattttt ccaaaggtgg attcctag                 1548

<210>  3

<211>  514
<212>  PRT
<213>  human

<400>  3

Met Gly His Asn Gly Ser Trp Ile Ser Pro Asn Ala Ser Glu Pro His
1               5                   10                  15

Asn Ala Ser Gly Ala Glu Ala Ala Gly Val Asn Arg Ser Ala Leu Gly
            20                  25                  30

Glu Phe Gly Glu Ala Gln Leu Tyr Arg Gln Phe Thr Thr Thr Val Gln
        35                  40                  45

Val Val Ile Phe Ile Gly Ser Leu Leu Glu Phe Gly Asn Met Glu Val
    50                  55                  60

Thr Arg Lys Leu Asp Lys Ser Arg Leu Pro Gly Ile Lys Phe Ile Lys
65                  70                  75                  80

Asn Leu Ala Cys Ser Gly Ile Cys Ala Ser Leu Val Cys Val Pro Phe
                85                  90                  95

Asp Ile Ile Leu Ser Thr Ser Pro His Cys Cys Trp Trp Ile Tyr Thr
            100                 105                 110

Met Leu Phe Cys Lys Val Val Lys Phe Leu His Lys Val Phe Cys Ser
        115                 120                 125

Val Thr Ile Leu Ser Phe Pro Ala Ile Ala Leu Asp Arg Tyr Tyr Ser
    130                 135                 140

Val Leu Tyr Pro Leu Glu Arg Lys Ile Ser Asp Ala Lys Ser Arg Glu
145                 150                 155                 160

Leu Val Met Tyr Ile Trp Ala His Ala Val Val Ala Ser Val Pro Val
                165                 170                 175

Phe Ala Val Thr Asn Val Ala Asp Ile Tyr Ala Thr Ser Thr Cys Thr
            180                 185                 190

Glu Val Trp Ser Asn Ser Leu Gly His Leu Val Tyr Val Leu Val Tyr
        195                 200                 205

Asn Ile Thr Thr Val Ile Val Pro Val Val Val Val Phe Leu Phe Leu
    210                 215                 220

Ile Leu Ile Arg Arg Ala Leu Ser Ala Ser Gln Lys Lys Lys Val Ile

225                 230                 235                 240

Ile Ala Ala Leu Arg Thr Pro Gln Asn Thr Ile Ser Ile Pro Tyr Ala
                245                 250                 255

Ser Gln Arg Glu Ala Glu Leu His Ala Thr Leu Leu Ser Met Val Met
            260                 265                 270

Val Phe Ile Leu Cys Ser Val Pro Tyr Ala Thr Leu Val Val Tyr Gln
        275                 280                 285

Thr Val Leu Asn Val Pro Asp Thr Ser Val Phe Leu Leu Leu Thr Ala
    290                 295                 300

Val Trp Leu Pro Lys Val Ser Leu Leu Ala Asn Pro Val Leu Phe Leu
305                 310                 315                 320

Thr Val Asn Lys Ser Val Arg Lys Cys Leu Ile Gly Thr Leu Val Gln
                325                 330                 335

Leu His His Arg Tyr Ser Arg Arg Asn Val Val Ser Thr Gly Ser Gly
            340                 345                 350

Met Ala Glu Ala Ser Leu Glu Pro Ser Ile Arg Ser Gly Ser Gln Leu
        355                 360                 365

Leu Glu Met Phe His Ile Gly Gln Gln Gln Ile Phe Lys Pro Thr Glu
    370                 375                 380

Asp Glu Glu Glu Ser Glu Ala Lys Tyr Ile Gly Ser Ala Asp Phe Gln
385                 390                 395                 400

Ala Lys Glu Ile Phe Ser Thr Cys Leu Glu Gly Glu Gln Gly Pro Gln
                405                 410                 415

Phe Ala Pro Ser Ala Pro Pro Leu Ser Thr Val Asp Ser Val Ser Gln
            420                 425                 430

Val Ala Pro Ala Ala Pro Val Glu Pro Glu Thr Phe Pro Asp Lys Tyr
        435                 440                 445

Ser Leu Gln Phe Gly Phe Gly Pro Phe Glu Leu Pro Pro Gln Trp Leu
    450                 455                 460

Ser Glu Thr Arg Asn Ser Lys Lys Arg Leu Leu Pro Pro Leu Gly Asn
465                 470                 475                 480

Thr Pro Glu Glu Leu Ile Gln Thr Lys Val Pro Lys Val Gly Arg Val
                485                 490                 495

Glu Arg Lys Met Ser Arg Asn Asn Lys Val Ser Ile Phe Pro Lys Val
            500                 505                 510

Asp Ser

<210>  4
<211>  1545
<212>  DNA
<213>  human

<400>  4
atgggacata acgggagctg gatctctcca aatgccagcg agccgcacaa cgcgtccggc      60
gccgaggctg cgggtgtgaa ccgcagcgcg ctcggggagt tcggcgaggc gcagctgtac     120
cgccagttca ccaccaccgt gcaggtcgtc atcttcatag gctcgctgct cgagtttggc     180
aacatggagg tcactagaaa acttgataag agcagactgc ctgggattaa gttcattaaa     240
aacctggcct gctcggggat ttgtgccagc ctggtctgtg tgcccttcga catcatcctc     300
agcaccagtc ctcactgttg ctggtggatc tacaccatgc tcttctgcaa ggtcgtcaaa     360
tttttgcaca aagtattctg ctctgtgacc atcctcagct tccctgctat tgctttggac     420
aggtactact cagtcctcta tccactggag aggaaaatat ctgatgccaa gtcccgtgaa     480
ctggtgatgt acatctgggc ccatgcagtg gtggccagtg tccctgtgtt tgcagtaacc     540
aatgtggctg acatctatgc cacgtccacc tgcacggaag tctggagcaa ctccttgggc     600
cacctggtgt acgttctggt gtataacatc accacggtca ttgtgcctgt ggtggtggtg     660
ttcctcttct tgatactgat ccgacgggcc ctgagtgcca gccagaagaa gaaggtcatc     720
atagcagcgc tccggacccc acagaacacc atctctattc cctatgcctc ccagcgggag     780
gccgagctgc acgccaccct gctctccatg gtgatggtct tcatcttgtg tagcgtgccc     840
tatgccaccc tggtcgtcta ccagactgtg ctcaatgtcc ctgacacttc cgtcttcttg     900
ctgctcactg ctgtttggct gcccaaagtc tccctgctgg caaaccctgt tctctttctt     960
actgtgaaca aatctgtccg caagtgcttg atagggaccc tggtgcaact acaccaccgg    1020
tacagtcgcc gtaatgtggt cagtacaggg agtggcatgg ctgaggccag cctggaaccc    1080
agcatacgct cgggtagcca gctcctggag atgttccaca ttgggcagca gcagatcttt    1140
aagcccacag aggatgagga agagagtgag gccaagtaca ttggctcagc tgacttccag    1200
gccaaggaga tatttagcac ctgcctggag ggagagcagg ggccacagtt tgcgccctct    1260
gccccacccc tgagcacagt ggactctgta tcccaggtgg caccggcagc ccctgtggaa    1320
cctgaaacat tccctgataa gtattccctg cagtttggct ttgggccttt tgagttgcct    1380
cctcagtggc tctcagagac ccgaaacagc aagaagcggc tgcttccccc cttgggcaac    1440
accccagaag agctgatcca gacaaaggtg cccaaggtag gcagggtgga gcggaagatg    1500
agcagaaaca ataaagtgag catttttcca aaggtggatt cctag                   1545

<210>  5
<211>  470
<212>  PRT
<213>  human

<400>  5
Met Pro Ala Pro Leu Leu Leu Ile Ser Arg Thr Leu Gly Asn Phe Met
1               5                   10                  15

Val Leu Trp Ser Thr Cys Arg Thr Thr Val Phe Lys Ser Val Thr Asn
            20                  25                  30

Arg Phe Ile Lys Asn Leu Ala Cys Ser Gly Ile Cys Ala Ser Leu Val
        35                  40                  45

Cys Val Pro Phe Asp Ile Ile Leu Ser Thr Ser Pro His Cys Cys Trp
    50                  55                  60

Trp Ile Tyr Thr Met Leu Phe Cys Lys Val Val Lys Phe Leu His Lys
65                  70                  75                  80

Val Phe Cys Ser Val Thr Ile Leu Ser Phe Pro Ala Ile Ala Leu Asp
                85                  90                  95

Arg Tyr Tyr Ser Val Leu Tyr Pro Leu Glu Arg Lys Ile Ser Asp Ala
            100                 105                 110

Lys Ser Arg Glu Leu Val Met Tyr Ile Trp Ala His Ala Val Val Ala
        115                 120                 125

Ser Val Pro Val Phe Ala Val Thr Asn Val Ala Asp Ile Tyr Ala Thr
    130                 135                 140

Ser Thr Cys Thr Glu Val Trp Ser Asn Ser Leu Gly His Leu Val Tyr
145                 150                 155                 160

Val Leu Val Tyr Asn Ile Thr Thr Val Ile Val Pro Val Val Val Val
                165                 170                 175

Phe Leu Phe Leu Ile Leu Ile Arg Arg Ala Leu Ser Ala Ser Gln Lys
            180                 185                 190

Lys Lys Val Ile Ile Ala Ala Leu Arg Thr Pro Gln Asn Thr Ile Ser
        195                 200                 205

Ile Pro Tyr Ala Ser Gln Arg Glu Ala Glu Leu His Ala Thr Leu Leu
    210                 215                 220

Ser Met Val Met Val Phe Ile Leu Cys Ser Val Pro Tyr Ala Thr Leu
225                 230                 235                 240

Val Val Tyr Gln Thr Val Leu Asn Val Pro Asp Thr Ser Val Phe Leu
                245                 250                 255

Leu Leu Thr Ala Val Trp Leu Pro Lys Val Ser Leu Leu Ala Asn Pro
            260                 265                 270

Val Leu Phe Leu Thr Val Asn Lys Ser Val Arg Lys Cys Leu Ile Gly
        275                 280                 285

Thr Leu Val Gln Leu His His Arg Tyr Ser Arg Arg Asn Val Val Ser
    290                 295                 300

Thr Gly Ser Gly Met Ala Glu Ala Ser Leu Glu Pro Ser Ile Arg Ser
305                 310                 315                 320

Gly Ser Gln Leu Leu Glu Met Phe His Ile Gly Gln Gln Gln Ile Phe
                325                 330                 335

Lys Pro Thr Glu Asp Glu Glu Glu Ser Glu Ala Lys Tyr Ile Gly Ser
            340                 345                 350

Ala Asp Phe Gln Ala Lys Glu Ile Phe Ser Thr Cys Leu Glu Gly Glu
        355                 360                 365

Gln Gly Pro Gln Phe Ala Pro Ser Ala Pro Pro Leu Ser Thr Val Asp
    370                 375                 380

Ser Val Ser Gln Val Ala Pro Ala Ala Pro Val Glu Pro Glu Thr Phe
385                 390                 395                 400

Pro Asp Lys Tyr Ser Leu Gln Phe Gly Phe Gly Pro Phe Glu Leu Pro
                405                 410                 415

Pro Gln Trp Leu Ser Glu Thr Arg Asn Ser Lys Lys Arg Leu Leu Pro
            420                 425                 430

Pro Leu Gly Asn Thr Pro Glu Glu Leu Ile Gln Thr Lys Val Pro Lys
        435                 440                 445

Val Gly Arg Val Glu Arg Lys Met Ser Arg Asn Asn Lys Val Ser Ile
    450                 455                 460

Phe Pro Lys Val Asp Ser
465                 470

<210>  6
<211>  1413
<212>  DNA
<213>  human

<400>  6

atgccagctc cactgcttct tatttctaga actttgggaa acttcatggt gttatggtca      60
acttgccgca caaccgtgtt caaatctgtc accaacaggt tcattaaaaa cctggcctgc     120
tcggggattt gtgccagcct ggtctgtgtg cccttcgaca tcatcctcag caccagtcct     180
cactgttgct ggtggatcta caccatgctc ttctgcaagg tcgtcaaatt tttgcacaaa     240
gtattctgct ctgtgaccat cctcagcttc cctgctattg ctttggacag gtactactca     300
gtcctctatc cactggagag gaaaatatct gatgccaagt cccgtgaact ggtgatgtac     360
atctgggccc atgcagtggt ggccagtgtc cctgtgtttg cagtaaccaa tgtggctgac     420
atctatgcca cgtccacctg cacggaagtc tggagcaact ccttgggcca cctggtgtac     480
gttctggtgt ataacatcac cacggtcatt gtgcctgtgg tggtggtgtt cctcttcttg     540
atactgatcc gacgggccct gagtgccagc cagaagaaga aggtcatcat agcagcgctc     600
cggaccccac agaacaccat ctctattccc tatgcctccc agcgggaggc cgagctgcac     660

gccaccctgc tctccatggt gatggtcttc atcttgtgta gcgtgcccta tgccaccctg 720
gtcgtctacc agactgtgct caatgtccct gacacttccg tcttcttgct gctcactgct 780
gtttggctgc ccaaagtctc cctgctggca aaccctgttc tctttcttac tgtgaacaaa 840
tctgtccgca agtgcttgat agggaccctg gtgcaactac accaccggta cagtcgccgt 900
aatgtggtca gtacagggag tggcatggct gaggccagcc tggaacccag catacgctcg 960
ggtagccagc tcctggagat gttccacatt gggcagcagc agatctttaa gcccacagag 1020
gatgaggaag agagtgaggc caagtacatt ggctcagctg acttccaggc caaggagata 1080
tttagcacct gcctggaggg agagcagggg ccacagtttg cgccctctgc cccacccctg 1140
agcacagtgg actctgtatc ccaggtggca ccggcagccc ctgtggaacc tgaaacattc 1200
cctgataagt attccctgca gtttggcttt gggccttttg agttgcctcc tcagtggctc 1260
tcagagaccc gaaacagcaa gaagcggctg cttcccccct tgggcaacac cccagaagag 1320
ctgatccaga caaaggtgcc caaggtaggc agggtggagc ggaagatgag cagaaacaat 1380
aaagtgagca tttttccaaa ggtggattcc tag 1413

<210> 7
<211> 515
<212> PRT
<213> mouse

<400> 7

Met Gly His Asn Ser Ser Trp Val Ser Pro Asn Thr Ser His Pro Arg
1               5                   10                  15

Asn Thr Ser Gly Ala Glu Ala Gly Ala Asn Leu Ser Ala Phe Gly Glu
            20                  25                  30

Leu Ser Glu Ala Gln Leu Tyr Arg Gln Phe Thr Thr Thr Val Gln Val
        35                  40                  45

Val Ile Phe Ile Gly Ser Leu Leu Gly Asn Phe Met Val Leu Trp Ser
    50                  55                  60

Thr Cys Arg Thr Thr Val Phe Lys Ser Val Thr Asn Arg Phe Ile Lys
65                  70                  75                  80

Asn Leu Ala Cys Ser Gly Ile Cys Ala Ser Val Val Cys Val Pro Phe
                85                  90                  95

Asp Ile Ile Leu Ser Ser Ser Pro His Cys Cys Trp Trp Ile Tyr Thr
            100                 105                 110

Met Leu Phe Cys Lys Val Leu Lys Phe Leu His Lys Val Phe Cys Ser
        115                 120                 125

Val Thr Val Leu Ser Phe Pro Ala Ile Ala Leu Asp Arg Tyr Tyr Ser
    130                 135                 140

Val Leu Tyr Pro Leu Glu Arg Lys Ile Ser Asp Ala Lys Ser Arg Glu
145                 150                 155                 160

Leu Val Met Tyr Ile Trp Ala His Ala Val Val Ala Ser Val Pro Val

                165                 170                 175

Phe Ala Val Thr Asn Val Ala Asp Ile Tyr Ala Met Ser Thr Cys Thr
            180                 185                 190

Glu Val Trp Ser Asn Ser Leu Gly His Leu Val Tyr Val Leu Ile Tyr
        195                 200                 205

Asn Val Thr Thr Val Ile Val Pro Val Ala Val Val Phe Leu Phe Leu
    210                 215                 220

Ile Leu Ile Arg Arg Ala Leu Ser Ala Ser Gln Lys Lys Lys Val Ile
225                 230                 235                 240

Ile Ala Ala Leu Arg Thr Pro Gln Asn Thr Ile Ser Ile Pro Tyr Ala
                245                 250                 255

Ser Gln Arg Glu Ala Glu Leu His Ala Thr Leu Leu Ser Met Val Thr
            260                 265                 270

Val Phe Ile Leu Cys Ser Val Pro Tyr Ala Thr Leu Val Val Tyr Gln
        275                 280                 285

Thr Val Leu Asn Val Pro Asn Thr Ser Val Phe Leu Leu Leu Thr Ala
    290                 295                 300

Ile Trp Leu Pro Lys Val Ser Leu Leu Ala Asn Pro Val Leu Phe Leu
305                 310                 315                 320

Thr Val Asn Lys Ser Val Arg Lys Cys Leu Val Gly Thr Leu Val Gln
                325                 330                 335

Leu His His Arg Tyr Ser Arg Arg Asn Val Val Ser Thr Gly Ser Gly
            340                 345                 350

Val Ala Glu Pro Ser Leu Glu Pro Ser Met Arg Ser Gly Ser Gln Leu
        355                 360                 365

Leu Glu Met Phe His Ile Gly Gln Gln Gln Ile Phe Lys Pro Ser Glu
    370                 375                 380

Asp Glu Glu Glu Ser Glu Ala Lys Tyr Leu Gly Ser Ala Asp Phe Gln
385                 390                 395                 400

Ala Lys Glu Val Leu Thr Ser Cys Pro Glu Gly Glu Gln Glu Pro Pro
                405                 410                 415

Gln Leu Ala Pro Ser Val Pro Pro Pro Gly Thr Val Asp Ser Glu Pro
            420                 425                 430

Arg Val Ser Pro Val Ala Pro Met Glu Ser Gly Ile Phe Pro Asp Lys
        435                 440                 445

Tyr Ser Leu Gln Phe Gly Phe Gly Pro Phe Glu Leu Pro Pro Gln Trp
    450                 455                 460

Leu Ser Glu Thr Arg Asn Ser Lys Lys Arg Leu Leu Pro Pro Leu Gly
465                 470                 475                 480

Asn Thr Pro Glu Glu Leu Ile Gln Thr Lys Val Pro Arg Val Asn Arg
                485                 490                 495

Val Glu Arg Lys Met Ser Arg Asn Asn Lys Val Ser Ile Phe Pro Lys
            500                 505                 510

Val Asp Ser
        515

<210>  8
<211>  1548
<212>  DNA
<213>  mouse

<400>  8

atggggcaca acagcagctg ggtgtcaccg aacaccagcc acccgcgcaa cacgtcgggc      60
gccgaggctg gcgccaacct cagcgcgttc ggggagctga gcgaggctca gctgtaccgc     120
cagttcacca ccaccgtgca ggtcgtcatc ttcattggct cgctactggg aaacttcatg     180
gtgttatggt caacttgccg cacaacagtg ttcaaatctg tcaccaacag gttcattaaa     240
aacctggcct gctccgggat ttgtgccagt gtggtctgtg tgcccttcga catcatcctg     300
agcagcagcc ctcactgttg ctggtggatc tatacgatgc tcttctgcaa ggtgctcaaa     360
ttcctacaca aggtgttctg ctctgtgact gtcctcagtt ttcctgctat tgccctggac     420
aggtactatt cagtccttta tcctctggag agaaaaatat ctgatgccaa gtctcgggag     480
ctagtgatgt acatctgggc tcatgcagtg gtggccagtg tccctgtgtt tgctgtgacc     540
aatgtcgctg acatctatgc tatgtccacc tgcacggaag tctggagcaa ctccttgggc     600
cacctggtgt atgttctgat ctacaatgtc accacagtca ttgtgcccgt ggctgtggta     660
tttctctttc tcatcctgat ccggagggcc ctgagtgcca gccagaagaa gaaagtcata     720
attgctgcac tgaggacccc acagaacacc atctctatcc cctatgcttc ccagcgagag     780
gcagagctac atgccaccct gctctccatg gtgacagttt tcattctctg cagtgtaccc     840
tatgccaccc tggttgtcta ccagactgtg ctcaatgtac ctaacacctc tgtcttcttg     900
cttctcacgg ccatttggct gcccaaagtc tctctgttgg ccaaccctgt gctcttctta     960
actgtgaaca aatcggtacg caagtgcttg gtggggaccc tggtgcagtt gcaccaccgg    1020
tacagtcgcc gcaatgtggt tagtacaggg agtggggtgg ctgagcccag ccttgagccc    1080
agcatgcgct ctggtagcca gctcttggag atgttccaca ttgggcagca acagatcttt    1140
aagccctcgg aggatgagga agaaagtgag gccaagtatc tcggctcagc cgatttccag    1200
gcaaaggagg tgctcacttc ctgcccagag ggcgagcagg agccaccaca gcttgcaccg    1260
tctgtgccac ctccaggcac agtggactct gaacctcggg tgtccccagt agctcccatg    1320
gaatctggga tatttcctga taagtattcc ctgcagtttg gctttgggcc ttttgagtta    1380
ccccctcagt ggctctctga gacccgaaat agcaagaaga ggctgcttcc cccactgggc    1440
aataccccag aagagctgat ccagacaaag gtgcccagag tgaacagggt ggaacggaag    1500
atgagcagaa acaataaagt gagcattttt ccaaaggtag actcctag                 1548

<210>  9
<211>  21
<212>  DNA
<213>  Artificial Sequence

<220>
<223>  Description of Artificial Sequence: Synthetic
       oligonucleotide

<220>
<223>  Description of Combined DNA/RNA Molecule: Synthetic
       oligonucleotide

<400>  9

gauauuuccu gauaaguaut t                                                21


<210>  10
<211>  21
<212>  RNA
<213>  Artificial Sequence

<220>
<223>  siRNA

<400>  10

auacuuauca ggaaauaucc c                                                21